# EUROPÄISCHE PATENTSCHRIFT

(11) **EP 1 127 567 B1**
(45) Veröffentlichungstag und Bekanntmachung des Hinweises auf die Patenterteilung: **01.09.2010**
(21) Anmeldenummer: 01101423.0
(22) Anmeldetag: 23.01.2001
(51) Int. Cl.: A61K 8/49, A61K 8/04, A61Q 17/04, A61Q 1/02, A61Q 19/00

(54) **Wässrige Dispersion wasserunlöslicher organischer UV-Filtersubstanzen**
Aqueous dispersions of water insoluble organic sunscreens
Dispersions aqueuses de filtres solaires organiques et insolubles dans l'eau

(30) Priorität: 17.02.2000 DE 10007116; 29.08.2000 DE 10042444
(43) Veröffentlichungstag der Anmeldung: 29.08.2001
(73) Patentinhaber: BASF SE, 67056 Ludwigshafen (DE)
(72) Erfinder: Heger, Robert, Dr., 69124 Heidelberg (DE); Auweter, Helmut, Dr., 67117 Limburgerhof (DE); Dausch, Wilma M., Dr., 67117 Limburgerhof (DE); Zwissler, Georg Konrad, Dr., 69115 Heidelberg (DE); Wünsch, Thomas, Dr., 67346 Speyer (DE)

(56) Entgegenhaltungen:
- EP-A- 0 761 201
- EP-A- 0 838 214
- EP-A- 0 893 119
- EP-A- 0 933 376
- WO-A-95/22959
- WO-A-97/03643
- DE-A- 2 354 756
- US-A- 5 025 004
- US-A- 5 133 908
- US-A- 5 360 559

## Beschreibung

Die Erfindung betrifft wäßrige Dispersionen schwer wasserlöslicher oder wasserunlöslicher organischer UV-Filtersubstanzen, deren Herstellung und Weiterverarbeitung zu Trockenpulvern sowie deren Verwendung als photostabile Lichtschutzmittel.

Die Qualität bzw. Lebensdauer vieler organischer Materialien, beispielsweise Kunststoffe und Lacke aber auch pharmazeutische und kosmetische Zubereitungen kann durch Einwirkung von Licht, insbesondere durch UV-Strahlen negativ beeinträchtigt werden. Diese Qualitätseinbußen zeigen sich bei Kunststoffen und Lacken häufig in Vergilbung, Verfärbung, Rißbildung oder Versprödung des Materials. Im Falle pharmazeutischer und kosmetischer Zubereitungen kann es durch den Einfluß von UV-Strahlen zum Abbau der in den Formulierungen enthaltenen Wirkstoffen kommen.

Die schädigende Wirkung des ultravioletten Teils der Sonnenstrahlung auf die Haut oder Haare, die im weitesten Sinne auch ein organisches Material darstellen, ist ebenfalls ein Problem, das immer mehr an Bedeutung gewinnt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, auf der Haut ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich u.a. um Derivate des 3-Benzylidencamphers, der 4-Aminobenzoesäure, der Zimtsäure, der Salicylsäure, des Benzophenons sowie des 2-Phenylbenzimidazols handelt.

Auch für den Bereich zwischen etwa 320 nm und etwa 400 nm, des sogenannten UVA-Bereich, ist es wichtig, Filtersubstanzen zur Verfügung zu haben, da dessen Strahlen Reaktionen bei lichtempfindlicher Haut hervorrufen können. Es ist erwiesen, daß UVA-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Zum Schutz gegen UVA-Strahlen werden Derivate des Dibenzoylmethans verwendet, deren Photostabilität jedoch nicht in ausreichendem Maße gegeben ist (Int. J. Cosm. Science 10, 53 (1988)).

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikaliche) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Ein anwendungstechnischer Nachteil vieler UV-Filter ist deren schlechte Löslichkeit in Wasser und/oder in Lipiden wodurch deren Verwendung beispielsweise in kosmetischen Formulierungen häufig eingeschränkt ist.

Ein weiterer Nachteil bei der Applikation einiger Lichtschutzmittel ist das Auftreten von Hautirritationen und Allergien aufgrund von zu hoher Hautdurchlässigkeit.

In GB-A-2 303 549 wird ein Mahlverfahren zur Herstellung von mikronisierten unlöslichen organischen UV-Absorbern in Gegenwart von Alkylpolyglykosiden beschrieben. Die so erhaltenen Mikronisate können in kosmetischen Lichtschutzzubereitungen eingearbeitet werden.

GB-A-2 286 774 beschreibt ebenfalls ein Mahlverfahren zur Mikronisierung unlöslicher organischer UV-Absorber.

Es war nun Aufgabe der vorliegenden Erfindung, UV-Lichtschutzmittel-Formulierungen bereitzustellen, die einen wirkungsvollen Schutz für organisches Material insbesondere für die menschliche Haut und/oder menschliche Haare vor UV-Strahlen mit sich bringen und die sich gut in wäßrigen und in lipophilen Systemen einarbeiten lassen.

Diese Aufgabe wurde gelöst durch wäßrige Dispersionen schwer wasserlöslicher oder wasserunlöslicher organischer UV-Filtersubstanzen, dadurch gekennzeichnet, daß sie mindestens eine schwer wasserlösliche oder wasserunlösliche organische UV-Filtersubstanz als kolloid-disperse Phase in amorpher oder teilamorpher Form enthalten, dadurch erhältlich, dass man
a) eine molekulardisperse Lösung mindestens einer schwer wasserlöslichen oder wasserunlöslichen organischen UV-Filtersubstanz in einem organischen Lösungsmittel herstellt,
b) diese Lösung mit einer wäßrigen Lösung mindestens eines Schutzkolloids versetzt, wobei die hydrophobe Phase der UV-Filtersubstanz als kolloid-disperse Phase entsteht und
c) die so erhaltene Dispersion vom organischen Lösungsmittel befreit;
worin die mindestens eine schwer wasserlösliche oder wasserunlösliche organische UV-Filtersubstanz ein 1,3,5-Triazinderivat der Formel I ist, 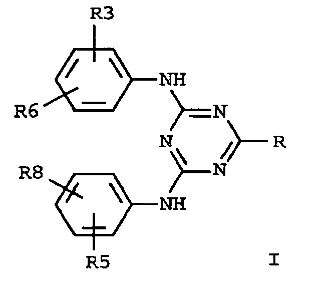 in der die Substituenten unabhängig voneinander folgende Bedeutung haben:
- R: Wasserstoff, Halogen, OH, C₁-C₂₀-Alkyl, C₁-C₂₀-Alkoxy, C₁-C₂₀- Alkoxyalkyl, C₁-C₂₀-Hydroxyalkoxy, NR¹R²,
oder einen Rest der Formel Ia 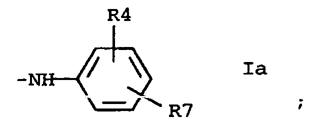
- R¹ und R²: Wasserstoff, C₁-C₂₀-Alkyl, gegebenenfalls mit einem oder mehreren C₁-C₄-Alkyl substituiertes C₆-C₁₂, Aryl, gegebenenfalls mit einem oder mehreren C₁-C₄-Alkyl substituiertes C₇-C₁₀ Aralkyl, gegebenenfalls mit einem oder mehreren C₁-C₄-Alkyl substituiertes Heteroaryl, C₅-C₈-Cycloalkyl;
- R³ bis R⁵: Wasserstoff, OH, NR⁹R¹⁰, C₁-C₂₀-Alkoxy, gegebenenfalls mit einem oder mehreren C₁-C₄-Alkyl substituiertes C₆-C₁₂ Aryl, gegebenenfalls mit einem oder mehreren C₁-C₄-Alkyl substituiertes C₇-C₁₀ Aralkyl, gegebenenfalls mit einem oder mehreren C₁-C₄-Alkyl substituiertes Heteroaryl, C₅-C₆- Cycloalkyl;
- R⁶ bis R⁸: Wasserstoff, C₁-C₂₀-Alkoxy, -C(=O)-R¹¹,-C(=O)-X-R¹², SO₂R¹³, CN;
- R⁹ bis R¹¹: Wasserstoff, C₁-C₂₀-Alkyl, gegebenenfalls mit einem oder mehreren C₁-C₄-Alkyl substituiertes C₆-C₁₂ Aryl, gegebenenfalls mit einem oder mehreren C₁-C₄-Alkyl substituiertes C₇-C₁₀ Aralkyl, gegebenenfalls mit einem oder mehreren C₁-C₄-Alkyl substituiertes Heteroaryl, C₅-C₈-Cycloalkyl;
- R¹²: Wasserstoff, C₁-C₂₀-Alkyl, gegebenenfalls mit einem oder mehreren C₁-C₄-Alkyl substituiertes C₆-C₁₂ Aryl, gegebenenfalls mit einem oder mehreren C₁-C₄-Alkyl substituiertes C₇-C₁₀ Aralkyl, gegebenenfalls mit einem oder mehreren C₁-C₄-Alkyl substituiertes Heteroaryl, C₅-C₈-Cycloalkyl oder einen Rest der Formel Sp-Sil;
- R¹³: C₁-C₂₀-Alkyl, C₅-C₆-Cycloalkyl, gegebenenfalls mit einem oder mehreren C₁-C₄-Alkyl substituiertes C₆-C₁₂ Aryl, gegebenenfalls mit einem oder mehreren C₁-C₄-Alkyl substituiertes C₇-C₁₀ Aralkyl, gegebenenfalls mit einem oder mehreren C₁-C₄-Alkyl substituiertes Heteroaryl;
- X: O, NR¹⁴;
- R¹⁴: Wasserstoff, C₁-C₂₀-Alkyl, gegebenenfalls mit einem oder mehreren C₁-C₄-Alkyl substituiertes C₆-C₁₂ Aryl, gegebenenfalls mit einem oder mehreren C₁-C₄-Alkyl substituiertes C₇-C₁₀ Aralkyl, gegebenenfalls mit einem oder mehreren C₁-C₄-Alkyl substituiertes Heteroaryl, C₅-C₈-Cycloalkyl;
- Sp: Spacer;
- Sil: Rest aus der Gruppe, bestehend aus Silane, Oligosiloxane und Polysiloxane.

Unter der Bezeichnung wäßrige Dispersionen sind im Rahmen der vorliegenden Erfindung sowohl wäßrige Suspensionen als auch Emulsionen zu verstehen. Bevorzugt sind wäßrigen Suspensionen zu nennen, bei denen die dispergierte Phase mindestens eine schwer wasserlösliche oder wasserunlösliche organische UV-Filtersubstanz als nanopartikuläre Teilchen enthält. Im Vordergrund der Erfindung stehen darüberhinaus auch die aus den obigen wäßrigen Suspensionen hergestellten Trockenpulver oder Emulsionen, bevorzugt Doppelemulsionen, insbesondere o/w/o-Emulsionen.

Unter schwer wasserlösliche organische UV-Filtersubstanzen sind in diesem Zusammenhang solche Verbindungen zu verstehen, deren Wasserlöslichkeit < 5 Gew.-%, bevorzugt < 1 Gew.-%, besonders bevorzugt < 0,5 Gew.-%, ganz besonders bevorzugt < 0,1 Gew.-% ist.

Die neuen Lichtschutzmittel-Formulierungen sind dadurch charakterisiert, daß sie mindestens eine schwer wasserlösliche oder wasserunlösliche organische UV-Filtersubstanz enthalten, deren amorpher Anteil im Bereich größer 10 %, bevorzugt größer 30 %, besonders bevorzugt im Bereich von 50 bis 100 %, ganz besonders bevorzugt im Bereich von 75 bis 99 % liegt. Die Bestimmung der Kristallinitätsrate der UV-Filtersubstanzen kann dabei beispielsweise durch Röntgenbeugungsmessungen erfolgen.

Der Gehalt an mindestens einer schwer wasserlöslichen oder wasserunlöslichen organischen UV-Filtersubstanz in den erfindungsgemäßen Lichtschutzmittel-Formulierungen liegt im Bereich von 0,1 bis 70 Gew.-%, bevorzugt im Bereich von 2 bis 40 Gew.-%, besonders bevorzugt im Bereich von 3 bis 30 Gew.-%, ganz besonders bevorzugt im Bereich von 5 bis 25 Gew.-%, bezogen auf die Trockenmasse der Formulierungen.

Die mittlere Teilchengröße der nanopartikulären Teilchen in der wäßrigen Dispersion liegt je nach Art der Formulierungsmethode im Bereich kleiner 10 µm, bevorzugt im Bereich kleiner 5 µm, besonders bevorzugt im Bereich von 0,01 bis 2 µm, ganz besonders bevorzugt im Bereich von 0,05 bis 1 µm.

Eine bevorzugte Form der erfindungsgemäßen, wäßrigen Dispersionen ist dadurch charakterisiert, daß die Teilchen der kolloid-dispersen Phase einen Kern/Schale-Aufbau haben, wobei der Kern mindestens eine schwer wasserlösliche oder wasserunlösliche organische UV-Filtersubstanz enthält. Die den Kern umhüllende Schale enthält im wesentlichen mindestens ein Schutzkolloid, vorzugsweise eine höhermolekulare Verbindung.

Aufgabe dieser Polymerhülle ist es, die Partikel in ihrem kolloidalen Zustand gegen heterogenes Teilchenwachstum (Aggregation, Flockung etc.) zu stabilisieren.

Es können für diesen Zweck ein oder mehrere Polymere eingesetzt werden. Die Molmassen der verwendeten Polymere liegen im Bereich von 1000 bis 10000000 g/mol, bevorzugt im Bereich von 1000 bis 1000000 g/mol. Grundsätzlich kommen alle für den Anwendungsbereich Pharma und Kosmetik geeigneten Polymere in Betracht.

Als polymere Stabilisatoren für die Hüllmatrix der Schale eignen sich erfindungsgemäß vorteilhafterweise wasserlösliche oder wasserquellbare Schutzkolloide wie beispielsweise Rinder-, Schweine- oder Fischgelatine, insbesondere sauer oder basisch abgebaute Gelatine mit Bloom-Zahlen im Bereich von 0 bis 250, ganz besonders bevorzugt Gelatine A 100, A 200, B 100 und B 200 sowie niedermolekulare, enzymatisch abgebaute Gelatinetypen mit der Bloom-Zahl 0 und Molekulargewichten von 15000 bis 25000 D wie zum Beispiel Collagel A und Gelitasol P (Firma Stoess, Eberbach) sowie Mischungen dieser Gelatine-Sorten sowie Stärke, Dextrin, Pektin, Gummiarabicum, Ligninsulfonate, Chitosan, Polystyrolsulfonat, Alginate, Kasein, Kaseinat, Methylcellulose, Carboxymethylcellulose, Hydroxypropylcellulose, Milchpulver, Dextran, Vollmilch oder Magermilch oder Mischungen dieser Schutzkolloide. Weiterhin eignen sich Homo- und Copolymere auf Basis von neutralen, kationischen oder anionischen Monomeren wie z.B. Ethylenoxid, Propylenoxid, Acrylsäure, Maleinsäureanhydrid, Milchsäure, N-Vinylpyrrolidon, Vinylacetat, α- und β-Asparaginsäure.

Die Wasserlöslichkeit bzw. Wasserquellbarkeit der o.g. Polymere ist dabei abhängig von der Temperatur, vom pH-Wert sowie von der Ionenstärke der Lösung.

Eine weitere, ebenfalls bevorzugte Form der erfindungsgemäßen wäßrigen Dispersion ist dadurch gekennzeichnet, daß die Teilchen der kolloid-dispersen Phase zusätzlich eine wasserunlösliche Polymermatrix enthalten, in die mindestens eine schwer wasserlösliche oder wasserunlösliche organische UV-Filtersubstanz eingebettet ist. Zur Verbesserung der kolloidalen Stabilität dieser wäßrigen Dispersion kann es vorteilhaft sein, wenn die dispergierten Teilchen ebenfalls von einem der o.g. Schutzkolloide umhüllt sind.

Die Matrixpolymere im Inneren der Teilchen tragen dazu bei, den Wirkstoff in seinem nicht kristallinen Zustand zu erhalten, sowie die kolloidalen Strukturen in Bezug auf homogenes Teilchenwachstum (Ostwald-Reifung) zu stabilisieren.

Als polymere Bestandteile, die sich im Kern der Partikel der erfindungsgemäßen Wirkstoffzubereitung befinden eignen sich prinzipiell alle Polymere, die in einem Temperaturbereich zwischen 0 und 240°C, einem Druckbereich zwischen und 100 bar einem pH-Bereich von 0 bis 14 oder Ionenstärken bis 10 mol/l nicht oder nur teilweise in Wasser oder wässrigen Lösungen oder Wasser-Lösungsmittelgemischen löslich sind.

Nicht oder nur teilweise löslich bedeutet in diesem Zusammenhang, daß der 2. Virialkoeffizient für das oder die Polymere in Wasser oder einem Gemisch aus Wasser und einem organischen Lösungsmittel Werte kleiner Null annehmen kann. (vgl. M.D. Lechner, "Makromolekulare Chemie", Birkhäuser Verlag, Basel, S. 170-175). Der 2. Virialkoeffizient, der eine Aussage über das Verhalten eines Polymers in einem Lösungsmittel(gemisch) macht, kann experimentell bestimmt werden, beispielsweise durch Lichtstreuungsmessung oder Bestimmung des osmotischen Drucks. Die Dimension dieses Koeffizienten ist (mol·l)/g².

Es können ein oder mehrere Polymere eingesetzt werden. Die Molmassen der verwendeten Polymere liegen im Bereich von 1000 bis 10000000 g/mol, bevorzugt im Bereich von 1000 bis 1000000 g/mol. Grundsätzlich kommen alle für den Anwendungsbereich Pharma und Kosmetik geeigneten Polymere in Betracht.

Von besonderem Interesse sind Polymere, die in organischen, mit Wasser mischbaren Lösungsmitteln löslich sind, und bei Temperaturen zwischen 0 und 240 °C nicht oder nur teilweise in Wasser oder wäßrigen Lösungen oder Wasser-Lösungsmittelgemischen löslich sind. Folgende Polymere sind beispielhaft genannt, ohne jedoch einschränkend zu sein:
Poly(vinylether) wie z.B. Poly(benzyloxyethylen), Poly(vinyl-acetale), Poly(vinylketone), Poly(allylalkohol), Poly(vinylester) wie z.B. Poly(vinylacetat), Poly(oxytetramethylen), Poly(glutardialdehyd), Poly(carbonate), Poly(ester), Poly(siloxane), Poly(amide), Poly(piperazine), Poly(anhydride) wie z.B. Poly-(metharylanhydrid), Gutta Percha, Celluloseether wie z.B. Methylzellulose (Substitutionsgrat: 3 bis 10 %), Ethylcellulose, Butylcellulose, Cellulose-Ester, wie z.B. Celluloseacetat oder Stärken. Insbesondere Copolymere und Blockcopolymere der Monomere der oben genannten Polymere. Weiterhin Copolymere und Blockcopolymere von Polyestern und Hydroxycarbonsäuren und linear- und Star-Polyethylenglycol, z.B. AB-, ABA- und ABC-Blockcopolymere aus einer Kombination aus L-Poly(lactid), Polyglycolid und/oder Polyethylenglycol, z.B Resomer 505, Resomer RG-756 oder Resomer RG-858 (Böhringer Ingelheim).

Von besonderem Interesse sind weiterhin Polymere, die bei Temperaturen zwischen 0 und 240°C in Wasser oder wäßrigen Lösungen oder Wasser-Lösungsmittelgemischen eine obere und/oder untere Mischungslücke besitzen, d.h. durch Erhöhung bzw. Erniedrigung der Temperatur können diese Polymere aus entsprechenden Lösungen ausgefällt werden. Folgende Polymere sind beispielhaft genannt, ohne jedoch einschränkend zu sein:
Poly(acrylamide), Poly(methacrylamide) wie z.B. Poly(N-isopropylacrylamid), Poly(N,N-dimethylacrylamid), Poly(N-(1,1-dimethyl-3-oxobutyl)acrylamid), Poly(methoxyethylen), Poly(vinylalkohole), acetylierte Poly(vinylalkohole), Poly(oxyethylen), Cellulose-Ether wie z.B. Methylcellulose (Substitutionsgrad: 20 bis 40 %), Isopropylcellulose, Cellulose-Ester, Stärken, modifizierte Stärken wie z.B Methylether-Stärke, Gummiarabicum, sowie Copolymere bzw. Blockcopolymere aus Monomeren der oben genannten Verbindungen. Insbesondere AB- oder ABA-Blockcopolymere auf der Basis Ethylenoxiod und Propylenoxid, z.B. Poloxamere wie Poloxamer 188 und Poloxamer 407.

Von besonderem Interesse sind weiterhin Polymere, die bei Temperaturen zwischen 0 und 240 °C in Wasser oder wäßrigen Lösungen oder Wasser-Lösungsmittelgemischen durch Variation des pH-Wertes oder der Ionenstärke aus entsprechenden Lösungen ausgefällt werden können. Folgende Polymere sind beispielhaft genannt, ohne jedoch einschränkend zu sein:
Alginate, Chitosan, Chitin, Schellak, Polyelektrolyte, Poly(acrylsäure) , Poly(methacrylsäure), Poly(methacrylester) mit mit sekundären tertiären oder quaternären Aminogruppen, insbesondere Copolymere oder Blockcopolymere auf der Basis verschiedener Acrylate, Methacrylate, Methacrylsäure, Acrylsäure, z.B. ein Copolymer aus Methacrylsäure/Methacrylsäureester (Gewichtsverhältnis MAS/MAE 1:1 oder 1:2) oder ein Copolymer aus Dimethylaminoethylmethacrylat und Methacrylsäureester im Gewichtsverhältnis 1:1 (Eudragit®-Typen).

Die Mengen der verschiedenen Komponenten werden erfindungsgemäß so gewählt, daß die Zubereitungen neben den oben genannten UV-Filtersubstanzen 0,1 bis 80 Gew.-%, bevorzugt 5 bis 70 Gew.-% besonders bevorzugt 10 bis 60 Gew.-%, ganz besonders bevorzugt 15 bis 35 Gew.-% eines oder mehrerer polymerer Schutzkolloide (Hüllpolymer) und gegebenenfalls 0,1 bis 80 Gew.-%, bevorzugt 0,5 bis 70 Gew.-%, besonders bevorzugt 1 bis 50 Gew.-%, ganz besonders bevorzugt 5 bis 35 Gew.-% eines oder mehrerer Matrixpolymere für den Kern enthalten. Die Gewichtsprozentangaben beziehen sich auf die Trockenmasse der Lichtschutzmittel-Formulierung.

Zusätzlich können die Zubereitungen noch niedermolekulare Stabilisatoren wie Antioxidantien und/oder Konservierungsmittel zum Schutz der UV-Filtersubstanzen enthalten. Geeignete Antioxidantien oder Konservierungsmittel sind beispielsweise α-Tocopherol, Ascorbinsäure, tert.-Butyl-hydroxytoluol, tert.-Butylhydroxyanisol, Lecithin, Ethoxyquin, Methylparaben, Propylparaben, Sorbinsäure oder Natriumbenzoat. Die Antioxidantien bzw. Konservierungsstoffe können in Mengen von 0,01 bis 50 Gew.-%, bevorzugt 0,1 bis 30 Gew.-%, besonders bevorzugt 0,5 bis 20 Gew.-%, ganz besonders bevorzugt 1 bis 10 Gew.-%, bezogen auf die Trockenmasse der Lichtschutzmittel-Formulierung.

Weiterhin können die Dispersionen noch Weichmacher zur Erhöhung der mechanischen Stabilität eines gegebenenfalls daraus hergestellten Trockenpulvers enthalten. Geeignete Weichmacher sind beispielsweise Zucker und Zuckeralkohole wie Saccharose, Glukose, Laktose, Invertzucker, Sorbit, Mannit, Xylit oder Glycerin. Bevorzugt wird als Weichmacher Laktose eingesetzt. Die Weichmacher können in Mengen von 0,1 bis 70 Gew.-%, bevorzugt 10 bis 60 Gew.-%, besonders bevorzugt 20 bis 50 Gew.-%, bezogen auf die Trockenmasse der Lichtschutzmittel-Formulierung enthalten sein.

Weiterhin können die Lichtschutzmittel-Formulierungen niedermolekulare oberflächenaktive Verbindungen (Emulgatoren) in einer Konzentration von 0,01 bis 70 Gew.-%, vorzugsweise 0,1 bis 50 Gew.-%, besonders bevorzugt 0,5 bis 20 Gew.-%, bezogen auf die Trockenmasse der Lichtschutzmittel-Formulierung enthalten. Als solche eignen sich vor allem amphiphile Verbindungen oder Gemische solcher Verbindungen. Grundsätzlich kommen alle Tenside mit einem HLB-Wert von 5 bis 20 in Betracht. Als entsprechende oberflächenaktive Substanzen kommen beispielsweise in Betracht: Ester langkettiger Fettsäuren mit Ascorbinsäure, Mono- und Diglyceride von Fettsäuren und deren Oxyethylierungsprodukte, Ester von Monofettsäureglyceriden mit Essigsäure, Zitronensäure, Milchsäure oder Diacetylweinsäure, Polyglycerinfettsäureester wie z.B. das Monostearat des Triglycerins, Sorbitanfettsäureester, Propylenglykolfettsäureester und Lecithin. Bevorzugt wird Ascorbylpalmitat eingesetzt.

Unter Umständen kann es auch vorteilhaft sein, zusätzlich ein physiologisch zugelassenes Öl wie beispielsweise Sesamöl, Maiskeimöl, Baumwollsaatöl, Sojabohnenöl oder Erdnußöl sowie kosmetische Öle, beispielsweise Paraffinöl, Glycerylstearat, Isopropylmyristat, Diisopropyladipat, 2-Ethylhexansäurecetylstearylester, hydriertes Polyisobuten, Vaseline, Caprylsäure/Caprinsäure-Triglyceride, mikrokristallines Wachs, Lanolin und Stearinsäure in einer Konzentration von 0,1 bis 500 Gew.-%, vorzugsweise 10 bis 300 Gew.-%, besonders bevorzugt 20 bis 100 Gew.-%, bezogen auf die eingesetzte(n) schwer wasserlösliche(n) oder wasserunlösliche(n) organische(n) UV-Filtersubstanz(en) zu verwenden.

Die erfindungsgemäße wäßrige Dispersion enthält mindestens ein 1,3,5-Triazinderivat der Formel I, 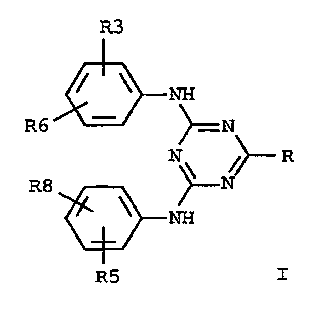 in der die Substituenten unabhängig voneinander folgende Bedeutung haben:
- R: Wasserstoff, Halogen, OH, C₁-C₂₀-Alkyl, C₁-C₂₀-Alkoxy, C₁-C₂₀-Alkoxyalkyl, C₁-C₂₀-Hydroxyalkoxy, NR¹R²,
oder einen Rest der Formel Ia 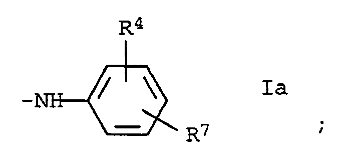
- R¹ und R²: Wasserstoff, C₁-C₂₀-Alkyl, gegebenenfalls mit einem oder mehreren C₁-C₄-Alkyl substituiertes C₆-C₁₂ Aryl, gegebenen- falls mit einem oder mehreren C₁-C₄-Alkyl substituiertes C₇-C₁₀ Aralkyl, gegebenenfalls mit einem oder mehreren C₁-C₄-Alkyl substituiertes Heteroaryl, C₅-C₈-Cycloalkyl;
- R³ bis R⁵: Wasserstoff, OH, NR⁹R¹⁰, C₁-C₂₀-Alkoxy, gegebenenfalls mit einem oder mehreren C₁-C₄-Alkyl substituiertes C₆-C₁₂ Aryl, gegebenenfalls mit einem oder mehreren C₁-C₄-Alkyl substi- tuiertes C₇-C₁₀ Aralkyl, gegebenenfalls mit einem oder mehreren C₁-C₄-Alkyl substituiertes Heteroaryl, C₅-C₈-Cyclo- alkyl;
- R⁶ bis R⁸: Wasserstoff, C₁-C₂₀-Alkoxy, -C(=O)-R¹¹,-C(=O)-X-R¹², SO₂R¹³, CN;
- R⁹ bis R¹¹: Wasserstoff, C₁-C₂₀-Alkyl, gegebenenfalls mit einem oder mehreren C₁-C₄-Alkyl substituiertes C₆-C₁₂ Aryl, gegebenen- falls mit einem oder mehreren C₁-C₄-Alkyl substituiertes C₇-C₁₀ Aralkyl, gegebenenfalls mit einem oder mehreren C₁-C₄-Alkyl substituiertes Heteroaryl, C₅-C₈-Cycloalkyl;
- R¹²: Wasserstoff, C₁-C₂₀-Alkyl, gegebenenfalls mit einem oder mehreren C₁-C₄-Alkyl substituiertes C₆-C₁₂ Aryl, gegebenen- falls mit einem oder mehreren C₁-C₄-Alkyl substituiertes C₇-C₁₀ Aralkyl, gegebenenfalls mit einem oder mehreren C₁-C₄-Alkyl substituiertes Heteroaryl, C₅-C₈-Cycloalkyl oder einen Rest der Formel Sp-Sil;
- R¹³: C₁-C₂₀-Alkyl, C₅-C₈-Cycloalkyl, gegebenenfalls mit einem oder mehreren C₁-C₄-Alkyl substituiertes C₆-C₁₂ Aryl, gegebenen- falls mit einem oder mehreren C₁-C₄-Alkyl substituiertes C₇-C₁₀ Aralkyl, gegebenenfalls mit einem oder mehreren C₁-C₄-Alkyl substituiertes Heteroaryl;
- X: O, NR¹⁴;
- R¹⁴: Wasserstoff, C₁-C₂₀-Alkyl, gegebenenfalls mit einem oder mehreren C₁-C₄-Alkyl substituiertes C₆-C₁₂ Aryl, gegebenen- falls mit einem oder mehreren C₁-C₄-Alkyl substituiertes C₇-C₁₀ Aralkyl, gegebenenfalls mit einem oder mehreren C₁-C₄-Alkyl substituiertes Heteroaryl, C₅-C₈-Cycloalkyl;
- Sp: Spacer;
- Sil: Rest der Gruppe, bestehend aus Silane, Oligosiloxane und Polysiloxane.

Als Alkylreste für R, R¹ und R² sowie R⁹ bis R¹⁴ seien verzweigte oder unverzweigte C₁-C₂₀-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, 2-Ethylhexyl, 1,1,3-Trimethylhexyl, 1,1,3,3-Tetramethylpentyl, n-Nonyl, n-Decyl, n-Undecyl, 1-Methylundecyl, n-Dodecyl, 1,1,3,3,5,5-Hexamethylhexyl, n-Tridecyl, n-Tetradecyl, n-Pentadecyl, n-Hexadecyl, n-Heptadecyl, n-Octadecyl, n-Nonadecyl oder n-Eicosyl genannt.

Halogen für R bedeutet Fluor, Brom, Jod oder vorzugsweise Chlor. Als Alkoxyreste für R sowie R³ bis R⁸ kommen gradkettige und verzweigte Reste mit 1 bis 20 C-Atomen, vorzugsweise mit 1 bis 12 C-Atomen, besonders bevorzugt mit 1 bis 8 C-Atomen in Betracht.

Beispielsweise sind zu nennen:

| | |
|---|---|
| Methoxy- | Ethoxy- |
| Isopropoxy- | n-Propoxy- |
| 1-Methylpropoxy- | n-Butoxy- |
| n-Pentoxy- | 2-Methylpropoxy- |
| 3-Methylbutoxy- | 1,1-Dimethylpropoxy- |
| 2,2-Dimethylpropoxy- | Hexoxy- |
| 1-Methyl-1-ethylpropoxy- | Heptoxy- |
| Octoxy- | 2-Ethylhexoxy- |

Als Hydroxyalkoxyreste für R kommen die o.g. Alkoxyreste mit zusätzlicher endständiger Hydroxylfunktion in Betracht.

Als Cycloalkylreste seien für R¹ bis R⁵ sowie R⁹ bis R¹⁴ bevorzugt verzweigte oder unverzweigte C₃-C₁₀-Cycloalkylreste wie Cyclopropyl, Cyclobutyl, Cyclopentyl, Cyclohexyl, Cycloheptyl, 1-Methylcyclopropyl, 1-Ethylcyclopropyl, 1-Propylcyclopropyl, 1-Butylcyclopropyl, 1-Pentylcyclopropyl, 1-Methyl-1-Butylcyclopropyl, 1,2-Dimethylcyclypropyl, 1-Methyl-2-Ethylcyclopropyl, Cyclooctyl, Cyclononyl oder Cyclodecyl genannt. Bevorzugt sind C₅-C₈-Cycloalkyl wie Cyclopentyl, Cycloheptyl, Cyclooctyl und insbesondere Cyclohexyl.

Die Cycloalkylreste können ggf. mit einem oder mehreren, z.B. 1 bis 3 Resten wie Halogen z.B. Fluor, Chlor oder Brom, Cyano, Nitro, Amino, C₁-C₄-Alkylamino, C₁-C₄-Dialkylamino, Hydroxy, C₁-C₄-Alkyl, C₁-C₄-Alkoxy oder anderen Resten substituiert sein oder 1 bis 3 Heteroatome wie Schwefel, Stickstoff, dessen freie Valenzen durch Wasserstoff oder C₁-C₄-Alkyl abgesättigt sein können oder Sauerstoff im Ring enthalten.

Als Beispiele für C₆-C₁₂-Aryl sind insbesondere Phenyl, Naphthyl und Biphenyl zu nennen.

Beispiele für C₇-C₁₀-Aralkyl sind Benzyl, Phenylethyl, α-Methylphenylethyl oder α,α-Dimethylbenzyl.

Heteroaryl-Reste sind vorteilhafterweise einfache oder kondensierte aromatische Ringsysteme mit einem oder mehreren heteroaromatische 3- bis 7-gliedrigen Ringen. Als Heteroatome können ein oder mehrere Stickstoff-, Schwefel- und/oder Sauerstoffatome im Ring oder Ringsystem enthalten sein.

Als Substituenten der o.g. Aryl-, Aralkyl- und Heteroarylreste kommen C₁-C₄-Alkylgruppen beispielsweise Methyl, Ethyl, n-Propyl, 1-Methylethyl, n-Butyl, 1-Methylpropyl-, 2-Methylpropyl, 1,1-Dimethylethyl in Frage.

Der Begriff Spacer für Sp bedeutet in diesem Zusammenhang eine bivalente verzweigte oder unverzweigte C₃-C₁₂-Alkylen- oder Alkenylenkette, die den Silan-, Oligosiloxan- oder Polysiloxanteil mit dem Triazinrest verknüpft.

Beispiele für eine C₃-C₁₂-Alkylenkette sind Propylen, 2-Methylpropylen, Butylen, Pentylen und Hexylen.

Beispiele für eine C₃-C₁₂-Alkenylenkette sind 2-Propen-2-ylen, 2-Methyl-3-propenylen, 3-Buten-3-ylen und 4-Penten-4-ylen.

Bevorzugte Spacer sind -(CH₂)₃-, -(CH₂)₄-, -(CH₂)₅-, -[CH(CH₃)]-(CH₂)-, -(CH₂)₂-CH=CH-, -C(=CH₂)-CH₂-, -C(=CH₂)-(CH₂)₂-O-(CH₂)₄-,-(CH₂)₄-O-(CH₂)₂-.

Der Begriff Silane steht in diesem Zusammenhang für einen Rest SiR¹⁵R¹⁶R¹⁷, in der R¹⁵, R¹⁶, R¹⁷ unabhängig voneinander für C₁-C₆-Alkyl, C₁-C₆-Alkoxy oder Phenyl stehen.

Als Beispiele seien zu nennen: Si(CH₂-CH₃)₃, Si(CH₂-CH₂-CH₃)₃, Si(isopropyl)₃, Si(ter.butyl)₃, Si(tert.butyl)(CH₃)₂, Si(CH₃)₂-(hexyl), Si(OCH₃)₃, Si(OEt)₃, SiPh₃.

Der Begriff Oligosiloxane bedeutet einen Rest aus der Gruppe der allgemeinen Formel, bestehend aus SiR¹⁸ₘ(oSiR¹⁸₃)ₙ mit m=0, 1 oder 2; n=3, 2 oder 1 und m+n=3, R¹⁸-[Si(R¹⁸)₂-O-]ᵣ-Si(R¹⁸)₂-A und R¹⁸-[Si(R¹⁸)₂-O-]ᵣ-Si(A)(R¹⁸)-O-Si(R¹⁸)₃, in denen A eine chemische Bindung oder einen Spacer und R¹⁸ einen C₁-C₆-Alkylrest oder Phenylrest bedeutet und r für Werte von 1 bis 9 steht.

Der Begriff Polysiloxane beinhaltet beispielsweise einen Rest aus der Gruppe der allgemeinen Formel, bestehend aus A-[Si(R¹⁹)₂-O]ₛ-Si(R¹⁹)₂-A oder (R¹⁹)₃-Si-[O-Si(R¹⁹)₂]]ₜ- [O-Si(R¹⁹) (A)]_{q}-O-Si(R¹⁹)₃, in denen A eine chemische Bindung oder einen Spacer und R¹⁹ einen C₁-C₆-Alkylrest oder Phenylrest bedeutet, s und t für Werte von 4 bis 250 und q für Werte von 1 bis 30 stehen.

Beispiele für Silanyl-Triazine, bei denen R¹² einen Rest der Formel Sp-Sil darstellt finden sich in EP-A-0 933 376.

Im Vordergrund stehen Triazinverbindungen der Formel Ib, 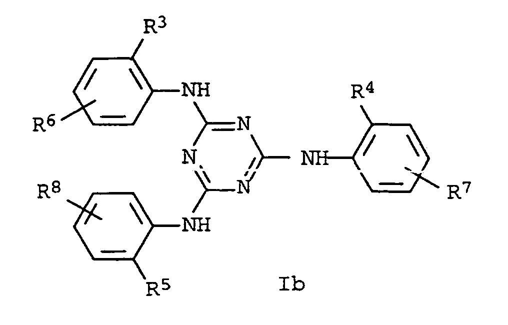 worin R³ bis R⁵ in ortho-Stellung zum Phenylaminorest des Triazins stehen.

Bevorzugt sind wäßrige Dispersion, enthaltend mindestens ein 1,3,5-Triazinderivat der Formel Ib, in der die Substituenten unabhängig voneinander folgende Bedeutung haben:
- R³ bis R⁵: Wasserstoff, OH;
- R⁶ bis R⁸: C₁-C₁₂-Alkoxy, -C(=O)-X-R¹²;
- X: O, NR¹⁴;
- R¹² und R¹⁴: Wasserstoff, C₄-C₈-Alkyl.

Ein besonders vorteilhafter UVB-Filter ist 2,4,6-Trianilino-p-(carbo-2'-ethyl-hexyl-1'-oxi)-1,3,5-triazin, der von der BASF Aktiengesellschaft unter der Warenbezeichnung Uvinul^{®} T150 vermarktet wird.

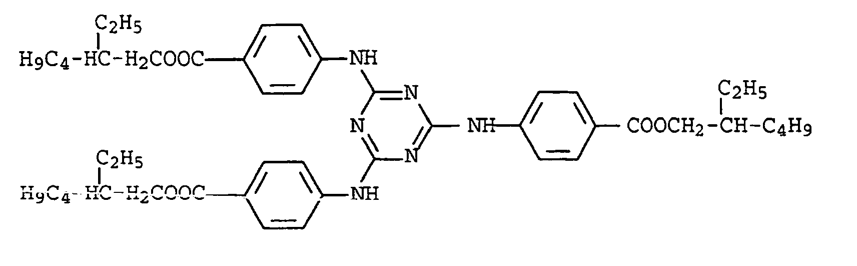

Uvinul^{®} T150 zeichnet sich durch gute UV-Absorptionseigenschaften mit einem außergewöhnlich hohen Extinktionskoeffizienten > 1500 bei 314 nm aus.

Der sphärische isotrope Charakter der in den erfindungsgemäßen Dispersionen enthaltenen Teilchen zeigt im Gegensatz zu gemahlenen anisotropen Teilchen kein strukturviskoses Verhalten. Eine Einarbeitung dieser Dispersion beispielsweise in kosmetische Formulierungen sowie die Anwendung dieser Formulierungen ist somit deutlich leichter.

Ferner konnte gezeigt werden, daß eine Sonnenschutzcreme auf Basis der erfindungsgemäßen Dispersion einen um den Faktor 4 höheren Sonnenschutzfaktor aufweist als eine analoge Lichtschutzmittelzubereitung, in der dieselbe Menge UV-Filtersubstanz molekular dispers vorgelegen hat.

Während gemahlene UV-Filtersubstanzen bei der Einarbeitung in Hautcremes verstärkt zu Teilchengrößenwachstum neigen, das zu einer Verschlechterung des Sonnenschutzfaktors und zu einem unangenehmen Hautgefühl führen kann, zeigen die erfindungsgemäßen Dispersionen aufgrund ihrer Matrix und Schutzkolloidstruktur derartige Tendenzen nicht.

Gegenstand der Erfindung ist auch ein Verfahren zur Herstellung einer wäßrigen Dispersion enthaltend mindestens eine schwer wasserlösliche oder wasserunlösliche organische UV-Filtersubstanz als kolloid-disperse Phase in amorpher oder teilamorpher Form, dadurch gekennzeichnet, daß man
a) eine molekulardisperse Lösung mindestens einer schwer wasserlöslichen oder wasserunlöslichen organischen UV-Filtersubstanz in einem organischen Lösungsmittel herstellt,
b) diese Lösung mit einer wäßrigen Lösung mindestens eines Schutzkolloids versetzt, wobei die hydrophobe Phase der UV-Filtersubstanz als kolloid-disperse Phase entsteht und
c) die so erhaltene Dispersion vom organischen Lösungsmittel befreit.

Als organisches Lösungsmittel im Verfahrensschritt a) wird vorteilhafterweise mindestens ein mit Wasser mischbares, organisches Lösungsmittel oder eine Mischung aus Wasser und mindestens einem mit Wasser mischbaren organischen Lösungsmittel verwendet.

Als bevorzugte Lösungsmittel sind vor allem solche wassermischbaren, thermisch stabilen, flüchtigen, nur Kohlenstoff, Wasserstoff und Sauerstoff enthaltenen Lösungsmittel wie Alkohole, Ether, Ester, Ketone und Acetale zu nennen. Zweckmäßig verwendet man solche Lösungsmittel, die mindestens zu 10 % wassermischbar sind, einen Siedepunkt unter 200°C aufweisen und/oder weniger als 10 Kohlenstoffe haben. Besonders bevorzugt werden Ethanol, n-Propanol, Isopropanol, 1,2-Butandiol-l-methylether, 1,2-Propandiol-1-n-propylether, Tetrahydrofuran oder Aceton verwendet.

Das Verfahren ist ferner dadurch gekennzeichnet, daß man der Lösung im Schritt a) gegebenenfalls weitere Zusatzstoffe, ausgewählt aus der Gruppe, bestehend aus Emulgatoren, Antioxidantien, Konservierungsmittel, wasserunlöslichen Polymeren und eßbaren Ölen zusetzt und/oder daß im Schritt b) die wäßrige Schutzkolloidlösung zusätzlich mindestens einen Weichmacher enthält. Es ist aber auch möglich, einen oder mehrere Emulgatoren der Schutzkolloidlösung zuzusetzen. Zur Beschreibung dieser Zusatzstoffe sei auf die bereits eingangs gemachten Erläuterungen hingewiesen.

In einer bevorzugten Ausführungsform des Verfahrens wird im Schritt a) die molekulardisperse Lösung mindestens einer schwer wasserlöslichen oder wasserunlöslichen organischen UV-Filtersubstanz bei Temperaturen größer 15°C, bevorzugt bei Temperaturen im Bereich von 50°C bis 240°C, besonders bevorzugt im Bereich von 140°C bis 180°C hergestellt und unmittelbar anschließend im Schritt b) mit der wäßrigen Lösung des Schutzkolloids versetzt, wobei sich eine Mischungstemperatur von etwa 35°C bis 120°C, besonders bevorzugt von 55°C bis 70°C einstellt.

Gegenstand der Erfindung ist weiterhin ein Verfahren zur Herstellung einer wäßrigen Dispersion mindestens einer schwer wasserlöslichen oder wasserunlöslichen organischen UV-Filtersubstanz, bei der die Teilchen der kolloid-dispersen Phase eine wasserunlösliche Polymermatrix enthalten, in die mindestens eine schwer wasserlösliche oder wasserunlösliche organische UV-Filtersubstanz eingebettet ist, dadurch gekennzeichnet, daß man
a) eine molekulardisperse Lösung mindestens einer schwer wasserlöslichen oder wasserunlöslichen organischen UV-Filtersubstanz und mindestens eines wasserunlöslichen Polymers in einem organischen Lösungsmittel herstellt,
b) diese Lösung mit einer wäßrigen Lösung mindestens eines Schutzkolloids versetzt, wobei die hydrophobe Phase der Mischung aus UV-Filtersubstanz und wasserunlöslichem Polymer als kolloid-disperse Phase entsteht und
c) die so erhaltene Dispersion vom organischen Lösungsmittel befreit.

Gemäß einer Ausführungsform des Verfahrens wird eine molekulardisperse Lösung der UV-Filtersubstanz in dem gewählten Lösungsmittel zusammen mit dem Polymer, daß in der Lichtschutzmittel-Zubereitung im Kern der Partikel liegen soll, hergestellt. Dieses Polymer hat die Eigenschaft, in einem bestimmten Temperatur-, pH-oder Salz-Bereich nicht oder nur teilweise in Wasser löslich zu sein.

Die Konzentration der so hergestellten Lichtschutzmittel-Polymer-Lösung beträgt im allgemeinen 10 bis 500 g mindestens einer schwer wasserlöslichen oder wasserunlöslichen UV-Filtersubstanz pro 1 kg Lösungsmittel und 0,01 bis 400 g Polymer, wobei das Polymer-Lichtschutzmittel-Gewichtsverhältnis zwischen 0.01 zu 1 und 5 zu 1 liegt. In einer bevorzugten Ausführungsform des Verfahrens wird der niedermolekulare Stabilisator direkt zu der Lichtschutzmittel-Polymer-Lösung gegeben.

In einem sich daran anschließenden Verfahrensschritt wird die Lichtschutzmittel-Polymer-Lösung mit Wasser oder bevorzugt einer wässrigen Lösung des polymeren Hüllmaterials vermischt. Die Konzentration des polymeren Hüllmaterials beträgt im allgemeinen 0,1 bis 200 g/l, vorzugsweise 1 bis 100 g/l.

Gemäß einer weiteren Ausführungsform des Verfahrens wird eine molekulardisperse Lösung der UV-Filtersubstanz in dem gewählten Lösungsmittel ohne dem Polymer, das in der Lichtschutzmittel-Zubereitung im Kern der Partikel liegen soll, hergestellt. Die Konzentration der so hergestellten Lichtschutzmittel-Lösung beträgt im allgemeinen 10 bis 500 g UV-Filtersubstanz pro 1 kg Lösungsmittel.

In einem sich daran anschließendem Verfahrensschritt wird diese Lösung mit einer wäßrigen molekularen Lösung des Polymers gemischt, das in der Lichtschutzmittel-Zubereitung im Kern der Partikel liegen soll. Die Konzentration der so hergestellten Polymer-Lösung beträgt im allgemeinen 0,01 bis 400 g Polymer. Dabei werden die Temperaturen, pH-Werte und Salzkonzentrationen der beiden zu vereinigenden Lösungen so gewählt, daß nach der Vereinigung der Lösungen die UV-Filtersubstanz und das Polymer in der vereinigten Lösung unlöslich sind. In einer bevorzugten Ausführungsform des Verfahrens wird der niedermolekulare Stabilisator direkt zu der Wirkstoff-Lösung gegeben.

In einem sich daran anschließenden Verfahrensschritt wird das Lichtschutzmittel-Polymer-Präzipitat mit einer wässrigen Lösung des polymeren Hüllmaterials vermischt. Die Konzentration des polymeren Hüllmaterials beträgt 0,1 bis 200 g/l, vorzugsweise 1 bis 100 g/l.

Der Mischvorgang kann diskontinuierlich oder, bevorzugt, kontinuierlich erfolgen. Als Folge des Mischvorgangs kommt es zu einer Präzipitation.

Welche Bedingungen bei der Durchführung des erfindungsgemäßen Verfahrens hinsichtlich Variation des Systems Wasser/organisches Lösungsmittel, der pH-Werte, der Temperaturen oder der Ionenstärken im konkreten Fall zu wählen sind, kann der Fachmann mit Hilfe des 2. Virialkoeffizienten durch einige einfache Vorversuche für das entsprechende Polymer ermitteln.

Gegenstand der Erfindung ist auch ein Verfahren zur Herstellung eines Trockenpulvers, enthaltend mindestens eine schwer wasserlösliche oder wasserunlösliche organische UV-Filtersubstanz als nanopartikuläre Teilchen in amorpher oder teilamorpher Form, dadurch gekennzeichnet, daß man eine der o.g. erfindungsgemäßen wäßrigen Dispersionen vom Wasser befreit und, gegebenenfalls in Gegenwart eines Überzugsmaterials, trocknet.

Die Überführung in ein Trockenpulver kann dabei beispielsweise durch Sprühtrocknung, Gefriertrocknung oder Trocknung im Wirbelbett, gegebenenfalls auch in Gegenwart eines Überzugsmaterials erfolgen. Als Überzugsmittel eignen sich u.a. Maisstärke, Kieselsäure oder auch Tricalciumphosphat.

Bei der Lyophilisation der erfindungsgemäßen Nanopartikel können kryoprotektive Substanzen wie z.B. Trehalose oder Polyvinylpyrrolidone zugesetzt werden.

Erfindungsgemäß lassen sich damit Trockenpulver erhalten, die ihre in der Primärdispersion gewonnen Eigenschaften nicht mehr verlieren. Das heißt, daß der amorphe Charakter der UV-Filtersubstanz sowie die Kern-Schale Struktur erhalten bleibt. Es ist weiterhin eine erfindungsgemäße Eigenschaft, das diese Dispersionen beim erneuten Auflösen mit einer Abweichung von 20 % bevorzugt < 15 % die gleiche Partikelgrößenverteilung zeigen, die sie als Primärdispersion besaßen.

Beansprucht werden auch pulverförmige Zubereitungen, erhältlich nach einem der o.g. Verfahren, enthaltend mindestens eine schwer wasserlöslichen oder wasserunlösliche organische UV-Filtersubstanz gemäß Anspruch 1 als nanopartikuläre Teilchen in amorpher oder teilamorpher Form.

Gegenstand der Erfindung ist ebenfalls ein Verfahren zur Herstellung einer mit Öl mischbaren Zubereitung in Form einer doppelten Dispersion, enthaltend mindestens eine schwer wasserlösliche oder wasserunlösliche organische UV-Filtersubstanz gemäß Anspruch 1 in amorpher oder teilamorpher Form, dadurch gekennzeichnet, daß man die eingangs beschriebenen wäßrigen Dispersionen in Öl emulgiert.

Dabei wird unter Verwendung eines Emulgators eine Wasser-in-Öl-Emulsion gebildet, in der die Wasserphase schutzkolloid-stabilisierte Nanoteilchen mindestens einer schwer wasserlöslichen oder wasserunlöslichen organischen UV-Filtersubstanz enthält. Als Emulgatoren kommen an sich bekannte W/O-Emulgatoren mit einem HLB-Wert kleiner 10, insbesondere von 2 bis 6 in Betracht (vgl. H.P. Fiedler, Lexikon der Hilfsstoffe für Pharmazie, Kosmetik und angrenzende Gebiete, 1996, S. 753 ff). Typische Vertreter dieser Emulgatorklasse sind Partialfettsäureester mehrwertiger Alkohole z.B. Glycerolmonostearat oder Mischungen aus Mono-, Di- und Triglyceriden, Partialfettsäureester des Sorbitans und/oder bevorzugt Fettsäureester des Polyglycerols wie beispielsweise Polyglycerol Polyricinoleat, die in einer Konzentration von 10 bis 1000 Gew.-%, vorzugsweise 100 bis 900 Gew.-%, besonders bevorzugt 400 bis 800 Gew.-%, bezogen auf die UV-Filtersubstanz(en), verwendet werden.

Das Dispersionsmittel kann sowohl synthetischen, mineralischen, pflanzlichen als auch tierischen Ursprungs sein. Typische Vertreter sind u.a. Sesamöl, Maiskeimöl, Baumwollsaatöl, Sojabohnenöl oder Erdnußöl, Ester mittelkettiger pflanzlicher Fettsäuren sowie Paraffinöl, Glycerylstearat, Isopropylmyristat, Diisopropyladipat, 2-Ethylhexansäurecetylstearylester, hydriertes Polyisobuten, Vaseline, Caprylsäure/Caprinsäure-Triglyceride, mikrokristallines Wachs, Lanolin und Stearinsäure. Die Menge des Dispersionsmittels beträgt im allgemeinen 30 bis 95, vorzugsweise 50 bis 80 Gew.-%, bezogen auf die Gesamtmasse der fertigen Emulsion.

Man kann das Emulgieren kontinuierlich oder diskontinuierlich durchführen.

Die physikalische Stabilität des doppelten Dispersionssystems, wie etwa die Sedimentationsstabilität, wird erreicht durch eine sehr gute Feinverteilung der Wasserphase in der Ölphase, z.B. durch intensive Behandlung mit einem Rotor/Stator-Dispergator bei Temperaturen von 20 bis 80, bevorzugt 40 bis 70°C oder mit einem Hochdruckhomogenisator wie einem APV Gaulin bzw. mit einem Höchstdruckhomogenisator wie dem Microfluidizer im Druckbereich von 700 bis 1000 bar. Die damit erreichbaren mittleren Durchmesser der wäßrig-dispersen Phase sind kleiner 500 µm, bevorzugt kleiner 100 µm, besonders bevorzugt kleiner 10 µm, insbesondere kleiner 1 µm.

Gegenstand der Erfindung sind auch flüssige, mit Öl mischbare Zubereitungen mindestens einer schwer wasserlöslichen oder wasserunlöslichen organischen UV-Filtersubstanz, erhältlich nach oben genanntem Verfahren, dadurch gekennzeichnet, daß sie als doppelte Dispersionssysteme eine wäßrig-disperse Phase mit einem Partikeldurchmesser kleiner 500 µm, in der schutzkolloid-stabilisierte Teilchen eines oder mehrerer schwer wasserlöslicher oder wasserunlöslicher organischer UV-Filtersubstanzen dispergiert vorliegen, in einem Öl als Dispersionsmittel enthalten.

Die erfindungsgemäßen Formulierungen - Dispersionen und daraus hergestellte Trockenpulver - eignen sich in hervorragender Weise zum Stabilisieren von organischem Material u.a. gegen die Einwirkung von Licht, Sauerstoff und Wärme. Sie werden dem zu stabilisierenden organischen Material in einer Konzentration von 0,01 bis 10 Gew.-%, bevorzugt 0,01 bis 5 Gew.-%, besonders bevorzugt von 0,02 bis 2 Gew.-%, bezogen auf das organische Material, vor, während oder nach seiner Herstellung zugesetzt.

Unter organischem Material sind beispielsweise photographische Aufzeichnungsmaterialien, insbesondere photographische Emulsionen oder Vorprodukte für Kunststoffe und Lacke, insbesondere jedoch Kunststoffe und Lacke selbst zu verstehen.

Unter organischem Material sind aber auch kosmetische Zubereitungen wie beispielsweise Cremes, Lotionen, Gele, Lippenstifte zu verstehen.

Gegenstand der vorliegenden Erfindung ist außerdem gegen die Einwirkung von Licht, Sauerstoff und Wärme stabilisiertes organisches Material, insbesondere Kunststoffe und Lacke, enthaltend 0,01 bis 10 Gew.-%, bevorzugt 0,01 bis 5 Gew.-%, besonders bevorzugt von 0,02 bis 2 Gew.-%, bezogen auf die Gesamtmenge des organischen Materials, eine oder mehrere schwer wasserlösliche oder wasserunlösliche organische UV-Filtersubstanzen in Form der erfindungsgemäßen Formulierungen.

Zur Vermischung der erfindungsgemäßen Formulierungen vor allem mit Kunststoffen können alle bekannten Vorrichtungen und Methoden zum Einmischen von Stabilisierungsmitteln oder anderen Zusätzen in Polymere angewandt werden.

Das durch die erfindungsgemäßen Formulierungen stabilisierte organische Material kann gegebenenfalls noch weitere Additive enthalten, z.B. Antioxidantien, Lichtstabilisierungsmittel, Metalldesaktivatoren, antistatische Mittel, flammhemmende Mittel, Pigmente und Füllstoffe.

Antioxidantien und Lichtstabilisatoren, die neben den erfindungsgemäßen Formulierungen zugesetzt werden können, sind z.B. Verbindungen auf der Basis sterisch gehinderter Phenole oder Schwefel oder Phosphor enthaltende Costabilisatoren.

Als derartige phenolische Antioxidationsmittel seien beispielsweise 2,6-Di-tert.-butyl-4-methylphenol, n-Octadecyl- β-(3,5-di-tert.-butyl-4-hydroxyphenyl)-propionat, 1,1,3-Tris-(2-methyl-4-hydroxy-5-tert.-butylphenyl)-butan, 1,3,5-Trimethyl-2,4,6-tris-(3,5-di-tert.-butyl-4-hydroxybenzyl)-benzol, 1,3,5-Tris-(3,5-di-tert.-butyl-4-hydroxybenzyl)-isocyanurat, 1,3,5-Tris-[ β-(3,5-di-tert.-butyl-4-hydroxyphenyl)-propionylethyl]isocyanurat, 1,3,5-Tris-(2,6-dimethyl-3-hydroxy-4-tert.-butylbenzyl)-isocyanurat und Pentaerythrit-tetrakis-[ β-(3,5-di-tert.-butyl-4-hydroxy-phenyl)-propionat] erwähnt.

Als phosphorhaltige Antioxidantien kommen beispielsweise Tris-(nonylphenyl)-phosphit, Distearylpentaerythritdiphosphit, Tris-(2,4-di-tert.-butyl-phenyl)-phosphit, Tris-(2-tert.-butyl-4-methylphenyl)-phosphit, Bis-(2,4-di-tert.-butylphenyl)-pentaerythritdiphosphit und Tetrakis-(2,4-di-tert.-butylphenyl)-4,4'-biphenylendiphosphit in Betracht.

Als Schwefel enthaltende Antioxidationsmittel seien beispielsweise Dilaurylthiodipropionat, Dimyristylthiodipropionat, Distearylthiodipropionat, Pentaerythrittetrakis-(β-laurylthiopropionat) und Pentaerythrittetrakis-(β-hexylthiopropionat) genannt.

Weitere Antioxidatien und Lichtstabilisatoren, die zusammen mit den erfindungsgemäßen Formulierungen verwendet werden können, sind z.B. 2-(2'-Hydroxyphenyl)-benztriazole, 2-Hydroxybenzophenone, Arylester von Hydroxybenzoesäuren, α-Cyanozimtsäurederivate, Benzimidazolcarbonsäureanilide, Nickelverbindungen oder Oxalsäuredianilide.

Eine besonders gute Stabilisierung erhält man, wenn zu den erfindungsgemäßen Formulierungen noch mindestens ein Lichtstabilisator aus der Verbindungsklasse der sterisch gehinderten Amine in üblicher Konzentration zugesetzt wird.

Als sterisch gehinderte Amine kommen z.B. in Betracht: Bis-(2,2,6,6-tetramethylpiperidyl)-sebacat, Bis-(1,2,2,6,6-pentamethylpiperidyl)-sebacat, das Kondensationsprodukt von 1-Hydroxyethyl-2,2,6,6-tetramethyl-4-hydroxypiperidin und Bernsteinsäure, das Kondensationsprodukt von N,N'-Di-(2,2,6,6-Tetramethylpiperidyl)-hexamethylendiamin und 4-tert.-Octylamino-2,6-dichlor-1,3,5-triazin, Tris-(2,2,6,6-Tetramethylpiperidyl)-nitrilotriacetat, Tetrakis-(2,2,6,6-tetramethyl-4-piperidyl)-1,2,3,4-butantetracarboxylat, 1,1'-(1,2-Ethandiyl)-bis-(3,3,5,5-tetramethylpiperazinon), die Kondensationsprodukte von 4-Amino-2,2,6,6-tetramethylpiperidinen und Tetramethylolacetylendiharnstoffen.

Als Kunststoffe, die durch die erfindungsgemäßen Verbindungen I stabilisiert werden können, seien beispielsweise genannt:
Polymere von Mono- und Diolefinen, wie z.B. Polyethylen niedriger oder hoher Dichte, Polypropylen, lineares Polybuten-1, Polyisopren, Polybutadien sowie Copolymerisate von Mono- oder Diolefinen oder Mischungen der genannten Polymeren;
Copolymerisate von Mono- oder Diolefinen mit anderen Vinylmonomeren, wie z.B. Ethylen-Alkylacrylat-Copolymere, Ethylen-Alkylmethacrylat-Copolymere, Ethylen-Vinylacetat-Copolymere oder Ethylen-Acrylsäure-Copolymere;
Polystyrol und Copolymere von Styrol oder α-Methylstyrol mit Dienen und/oder Acrylderivaten, wie z.B. Styrol-Butadien, Styrol-Acrylnitril (SAN), Styrol-Ethylmethacrylat, Styrol-Butadien-Ethylacrylat, Styrol-Acrylnitril-Methacrylat, Acrylnitril-Butadien-Styrol (ABS) oder Methylmethacrylat-Butadien-Styrol (MBS);
halogenhaltige Polymere, wie z.B. Polyvinylchlorid, Polyvinylfluorid, Polyvinylidenfluorid sowie deren Copolymere;
Polymere, die sich von α,β-ungesättigten Säuren und deren Derivaten ableiten, wie Polyacrylate, Polymethacrylate, Polyacrylamide und Polyacrylnitrile;
Polymere, die sich von ungesättigten Alkoholen und Aminen bzw. von deren Acylderivaten oder Acetalen ableiten, z.B. Polyvinylalkohol und Polyvinylacetat;
Polyurethane, Polyamide, Polyharnstoffe, Polyester, Polycarbonate, Polysulfonate, Polyethersulfone und Polyetherketone.

Weiterhin können mit den erfindungsgemäßen Formulierungen Lacküberzüge stabilisiert werden, z.B. Industrielackierungen. Unter diesen sind Einbrennlackierungen, unter diesen wiederum Fahrzeuglackierungen, vorzugsweise Zweischichtlackierungen, besonders hervorzuheben.

Die Formulierungen können in fester oder flüssiger Form dem Lack zugesetzt werden. Ihre gute Löslichkeit in Lacksystemen ist dabei von besonderem Vorteil.

Auch bei der Verwendung als Stabilisatoren in Lacken können die bereits aufgeführten zusätzlichen Additive, insbesondere Antioxidantien und Lichtstabilisatoren, mitverwendet werden.

Die erfindungsgemäßen Formulierungen zeichnen sich durch eine gute Verträglichkeit mit den üblichen Kunststoffarten und durch eine gute Löslichkeit in den üblichen Lacksystemen und in den üblichen kosmetischen Ölen aus. Sie haben in der Regel keine oder nur eine sehr geringe Eigenfarbe, sind bei den üblichen Kunststoff- und Lack-Verarbeitungstemperaturen stabil und nicht flüchtig, zeigen eine nur geringe Migrationsneigung und bewirken vor allen Dingen eine lange Schutzdauer der mit ihnen behandelten organischen Materialien.

Weiterhin eignen sich die erfindungsgemäßen Lichtschutzmittel Formulierungen auch als photostabile UV-Filter in kosmetischen und pharmazeutischen Zubereitungen zum Schutz der menschlichen Haut oder menschlicher Haare gegen Sonnenstrahlen aber auch gegen künstliches Licht, welches hohe UV-Anteile aufweist, allein oder zusammen mit für kosmetische oder pharmazeutische Zubereitungen bekannten, im UV-Bereich absorbierenden Verbindungen. Unter organischen Materialien sind im weitesten Sinne somit auch die menschliche Haut und menschliche Haare zu verstehen. Die kosmetischen und pharmazeutischen Zubereitungen als solche werden zugleich natürlich auch stabilisiert, um möglichst lange wirksam zu bleiben.

Demgemäß sind auch Gegenstand der vorliegenden Erfindung Lichtschutzmittel enthaltende kosmetische und pharmazeutische Zubereitungen zum Schutz der menschlichen Haut oder menschlicher Haare gegen UV-Licht im Bereich von 280 bis 400 nm, dadurch gekennzeichnet, daß sie in einem kosmetisch oder pharmazeutisch geeigneten Träger als photostabile UV-Filter wirksame Mengen einer Formulierung schwer wasserlöslicher oder wasserunlöslicher organischer UV-Filtersubstanzen in amorpher oder teilamorpher Form - allein oder zusammen mit an sich für kosmetische und pharmazeutische Zubereitungen bekannten, im UV-A- und UV-B-Bereich absorbierenden Verbindungen - enthalten, wobei es sich bei den Formulierungen um die eingangs genannten erfindungsgemäßen wäßrigen Dispersionen oder den daraus hergestellten O/W/O Emulsionen oder Trockenpulvern handelt.

Die Menge an schwer wasserlöslicher oder wasserunlöslicher organischer UV-Filtersubstanz in Form der erfindungsgemäßen Formulierungen, die in den kosmetischen und pharmazeutischen Zubereitungen eingesetzt wird, liegt im Bereich von 0,05 bis 20 Gew.-%, bevorzugt 0,1 bis 10 Gew.-%, besonders bevorzugt im Bereich von 1 bis 7 Gew.-%, bezogen auf die gesamte Menge der kosmetischen und pharmazeutischen Zubereitung.

Die Lichtschutzmittel enthaltenden kosmetischen und pharmazeutischen Zubereitungen sind in der Regel auf der Basis eines Trägers, der mindestens eine Ölphase enthält. Es sind aber auch Zubereitungen allein auf wäßriger Basis möglich. Demgemäß kommen Öle, Öl-in-Wasser- und Wasser-in-Öl-Emulsionen, Cremes und Pasten, Lippenschutzstiftmassen oder fettfreie Gele in Betracht.

Als Emulsionen kommen u.a. auch O/W-Makroemulsionen, O/W-Mikroemulsionen oder O/W/O-Emulsionen mit in dispergierter Form vorliegenden aminosubstituierten Hydroxybenzophenonen der Formel I in Frage, wobei die Emulsionen durch Phaseninversionstechnologie, gemäß DE-A-197 26 121 erhältlich sind.

Übliche kosmetische Hilfsstoffe, die als Zusätze in Betracht kommen können, sind z.B. Co-Emulgatoren, Fette und Wachse, Stabilisatoren, Verdickungsmittel, biogene Wirkstoffe, Filmbildner, Duftstoffe, Farbstoffe, Perlglanzmittel, Konservierungsmittel, Pigmente, Elektrolyte (z.B. Magnesiumsulfat) und pH-Regulatoren. Als Co-Emulgatoren kommen vorzugsweise bekannte W/O- und daneben auch O/W-Emulgatoren wie etwa Polyglycerinester, Sorbitanester oder teilveresterte Glyceride in Betracht. Typische Beispiele für Fette sind Glyceride; als Wachse sind u.a. Bienenwachs, Paraffinwachs oder Mikrowachse gegebenenfalls in Kombination mit hydrophilen Wachsen zu nennen. Als Stabilisatoren können Metallsalze von Fettsäuren wie z.B. Magnesium-, Aluminium- und/oder Zinkstearat eingesetzt werden. Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polyacrylate, Polyvinylalkohol und Polyvinylpyrrolidon. Unter biogenen Wirkstoffen sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als Konservierungsmittel eignen sich beispielsweise Formaldehydlösung, p-Hydroxybenzoat oder Sorbinsäure. Als Perlglanzmittel kommen beispielsweise Glycoldistearinsäureester wie Ethylenglycoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkoimmission der Deutschen Forschungsgemeinschaft, veröffentlicht im Verlag Chemie, Weinheim, 1984, zusammengestellt sind. Diese Farbstoffe werden üblicherweise in Konzentration von 0,001 bis 0,1 Gew.-%, bezogen auf die gesamte Mischung, eingesetzt.

Ein zusätzlicher Gehalt an Antioxidantien ist im allgemeinen bevorzugt. So können als günstige Antioxidantien alle für kosmetische und/oder dermatologische Anwendungen geeigneten oder gebräuchlichen Antioxidantien verwendet werden.

Vorteilhafterweise werden die Antioxidantien gewählt aus der Gruppe, bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thiorodoxin, Glutathion, Cystein, Cystin, Cystamin und deren Glycosyl-, N-Acetyl-, Methyl-, Ethyl-, Propyl-, Amyl-, Butyl-, und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäuure und deren Derivate (Ester, Ether, Peptide, Lipide, Nukleotide, Nukleoside und Salze) sowie Sulfoximinverbindungen (z.B. Buthioninsulfoximine, Homocysteinsulfoximine, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Biliburin, Biliverdin, EDTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und deren Derivate (z.B. Ascorbylpalmitat, Mg-Ascorbylphosphat, Ascorbylacetat), Tocopherol und Derivate (z.B. Vitamin-E-Acetat, Tocotrienol), Vitamin A und Derivate (Vitamin-A-Palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, Trans-Stilbenoxid).

Die Menge der vorgenannten Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 1 bis 10 Gew.-%, bezogen auf das Gesamtgewicht der Zubereitung.

Sofern Vitamin E und/oder dessen Derivate das oder die Antioxidantien darstellen, ist es vorteilhaft, deren jeweilige Konzentration aus dem Bereich von 0,001 bis 10 Gew.-%, bezogen auf das Gesamtgewicht der Formulierung, zu wählen.

Sofern Vitamin A und/oder dessen Derivate bzw. Carotinoide das oder die Antioxidantien darstellen, ist es vorteilhaft, deren jeweilige Konzentration aus dem Bereich von 0,001 bis 10 Gew.-%, bezogen auf das Gesamtgewicht der Formulierung, zu wählen.

Übliche Ölkomponenten in der Kosmetik sind beispielsweise Paraffinöl, Glycerylstearat, Isopropylmyristat, Diisopropyladipat, 2-Ethylhexansäurecetylstearylester, hydriertes Polyisobuten, Vaseline, Caprylsäure/Caprinsäure-Triglyceride, mikrokristallines Wachs, Lanolin und Stearinsäure.

Der Gesamtanteil der Hilfs- und Zusatzstoffe kann 1 bis 80, vorzugsweise 6 bis 40 Gew.-% und der nicht wäßrige Anteil ("Aktivsubstanz") 20 bis 80, vorzugsweise 30 bis 70 Gew.-% - bezogen auf die Mittel - betragen. Die Herstellung der Mittel kann in an sich bekannter Weise, d.h. beispielsweise durch Heiß-, Kalt-, Heiß-Heiß/Kalt- bzw. PIT-Emulgierung erfolgen. Hierbei handelt es sich um ein rein mechanisches Verfahren, eine chemische Reaktion findet nicht statt.

Solche Sonnenschutzpräparate können demgemäß in flüssiger, pastöser oder fester Form vorliegen, beispielsweise als Wasser-in-Öl-Cremes, Öl-in-Wasser-Cremes und -Lotionen, Aerosol-Schaumcremes, Gele, Öle, Fettstifte, Puder, Sprays oder alkoholischwäßrige Lotionen.

Schließlich können weitere an sich bekannte im UV-Bereich absorbierenden Substanzen mitverwendet werden, sofern sie im Gesamtsystem der erfindungsgemäß zu verwendenden Kombination aus UV-Filtern stabil sind.

Der größte Teil der Lichtschutzmittel in den zum Schutz der menschlichen Epidermis dienenden kosmetischen und pharmazeutischen Zubereitungen besteht aus Verbindungen, die UV-Licht im UV-B-Bereich absorbieren d.h. im Bereich von 280 bis 320 nm. Beispielsweise beträgt der Anteil der erfindungsgemäß zu verwendenden UV-A-Absorber 10 bis 90 Gew.-%, bevorzugt 20 bis 50 Gew.-% bezogen auf die Gesamtmenge von UV-B und UV-A absorbierenden Substanzen.

Als UV-Filtersubstanzen, die in Kombination mit den erfindungsgemäß zu verwendenden Formulierungen angewandt werden, kommen beliebige UV-A- und UV-B-Filtersubstanzen in Betracht. Beispielsweise sind zu nennen:

| Nr. | Stoff | CAS-Nr. (=Säure) |
|---|---|---|
| 1 | 4-Aminobenzoesäure | 150-13-0 |
| 2 | 3-(4'Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxy-ethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfon-(Sulisobenzonum) und das Natriumsalz | 4065-45-6 |
| 13 | 3-(4'-Sulfo)benzyliden-bornan-2-on und Salze | 58030-58-6 |
| 14 | 3 Benzylidenbornan-2-on | 16087-24-8 |
| 15 | 1-(4'-Isopropylphenyl)-3-phenylpropan-1,3-dion | 63260-25-9 |
| 16 | 4-Isopropylbenzylsalicylat | 94134-93-7 |
| 17 | 2,4,6-Trianilin-(o-carbo-2'-ethylhexyl-1'-oxy)-1,3,5-triazin | 88122-99-0 |
| 18 | 3-Imidazol-4-yl-acrylsäure und ihr Ethylester | 104-98-3 |
| 19 | 2-Cyano-3,3-diphenylacrylsäureethylester | 5232-99.-5 |
| 20 | 2-Cyano-3,3-diphenylacrylsäure-2'-ethylhexyl-ester | 6197-30-4 |
| 21 | Menthyl-o-aminobenzoate oder: 5-Methyl-2-(1-methylethyl)-2-aminobenzoate | 134-09-8 |
| 22 | Glyceryl p-aminobenzoat oder: 4-Aminobenzoesäure-1-glyceryl-ester | 136-44-7 |
| 23 | 2,2'-Dihydroxy-4-methoxybenzophenon (Dioxybenzone) | 231-53-3 |
| 24 | 2-Hydroxy-4-methoxy-4-methylbenzophenon (Mexonon) | 1641-17-4 |
| 25 | Triethanolamin Salicylat | 2174-16-5 |
| 26 | Dimethoxyphenylglyoxalsäure oder: 3,4-dimethoxy-phenyl-glyoxal-saures Natrium | 4732-70-1 |
| 27 | 3-(4'Sulfo)benzyliden-bornan-2-on und seine Salze | 56039-58-8 |
| 28 | 4-tert.-Butyl-4'-methoxy-dibenzoylmethan | 70356-09-1 |
| 29 | 2,2',4,4'-Tetrahydroxybenzophenon | 131-55-5 |
| 30 | 2,2'-Methylen-bis-[6(2H-benzotriazol-2-yl)-4-(1,1,3,3,-tetramethylbutyl)phenol] | 103597-45-1 |
| 31 | 2,2'-(1,4-Phenylen)-bis-1H-benzimidazol-4,6-disulfonsäure, Na-Salz | 180898-37-7 |
| 32 | 2,4-bis-[4-(2-Ethylhexyloxy)-2-hydroxy]phenyl-6-(4-methoxyphenyl)-(1,3,5)-triazin | 187393-00-6 |
| 33 | 3-(4-Methylbenzyliden)-campher | 36861-47-9 |
| 34 | 4-Bis(polyethoxy)paraaminobenzoesäurepoly-ethoxyethylester | 113010-52-9 |
| 35 | 2,4-Dihydroxybenzophenon | 131-56-6 |
| 36 | 2,2'-Dihydroxy-4,4'-dimethoxybenzophenon-5,5'-dinatriumsulfonat | 3121-60-6 |

Auch polymere oder polymergebundene Filtersubstanzen können erfindungsgemäß verwendet werden.

Die erfindungsgemäßen kosmetischen und dermatologischen Zubereitungen können vorteilhafterweise außerdem anorganische Pigmente auf Basis von Metalloxiden und/oder anderen in Wasser schwerlöslichen oder unlöslichen Metallverbindungen, insbesondere der Oxide des Titans (TiO₂), Zinks (ZnO), Eisens (z.B. Fe₂O₃), Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle sowie Abmischungen aus solchen Oxiden enthalten. Besonders bevorzugt handelt es sich um Pigmente auf der Basis TiO₂ und ZnO.

Es ist besonders vorteilhaft im Sinne der vorliegenden Erfindung, wenngleich nicht zwingend, wenn die anorganischen Pigmente in hydrophober Form vorliegen, d.h. daß sie oberflächlich wasserabweisend behandelt sind. Diese Oberflächenbehandlung kann darin bestehen, daß die Pigmente nach an sich bekannter Weise, wie in DE-A-33 14 742 beschrieben, mit einer dünnen hydrophoben Schicht versehen sind.

Zum Schutz menschlicher Haare vor UV-Strahlen können die erfindungsgemäßen Lichtschutzmittel-Formulierungen in Shampoos, Lotionen, Gelen, Haarsprays, Aerosol-Schaumcremes oder Emulsionen in Konzentrationen von 0,1 bis 10 Gew.-%, bevorzugt 1 bis 7 Gew.-% eingearbeitet werden. Die jeweiligen Formulierungen können dabei u.a. zum Waschen, Färben sowie zum Frisieren der Haare verwendet werden.

Die erfindungsgemäß zu verwendenden Formulierungen zeichnen sich in der Regel durch ein besonders hohes Absorptionsvermögen im Bereich der UV-A-Strahlung mit scharfer Bandenstruktur aus. Weiterhin sind sie gut in kosmetischen Ölen löslich und lassen sich leicht in kosmetische Formulierungen einarbeiten. Die mit den Formulierungen hergestellten Emulsionen zeichnen sich besonders durch ihre hohe Stabilität, die Formulierungen I selber durch ihre hohe Photostabilität aus, und die damit hergestellten Zubereitungen durch ihr angenehmes Hautgefühl aus.

Die UV-Filterwirkung der erfindungsgemäßen Formulierungen kann auch zur Stabilisierung von Wirk- und Hilfsstoffen in kosmetischen und pharmazeutischen Formulierungen ausgenutzt werden.

Die erfindungsgemäßen Zubereitungen zeichnen sich durch ein besonders hohes Absorptionsvermögen im Bereich der UV-B-Strahlung mit scharfer Bandenstruktur und hohen Lichtschutzfaktoren aus. Insbesondere der hohe Lichtschutzfaktor der Zubereitungen, der bereits bei niedrigen Konzentrationen an UV-absorbierenden Wirkstoffen gemessen wurde, war überraschend.

Die folgenden Beispiele sollen die vorliegende Erfindung verdeutlichen, ohne sie einzuschränken.

### Beispiel 1

### Herstellung eines Uvinul^{®} T 150-haltigen Trockenpulvers mit einem Wirkstoffgehalt von 20 Gew.-%

### a) Herstellung der wäßrigen Dispersion

10 g Uvinul^{®} T 150 wurden in 216 g Aceton bei Raumtempertur molekular-dispers gelöst. Zur Ausfällung des Uvinul^{®} T 150 in kolloid-disperser Form wurde die Lösung bei 240°C einer Mischkammer zugeführt. Dort erfolgte die Vermischung mit einer mittels 1 N NaOH auf pH 9,1 eingestellten wäßrigen Lösung von 16,88 g Gelatine B 100 Bloom und 11,25 g Lactose in 1500 ml VE-Wasser. Der gesamte Prozeß erfolgte unter Druckbegrenzung auf 40 bar, um eine Verdampfung des Lösemittels zu verhindern. Nach dem Mischen wurde eine kolloid-disperse Uvinul^{®} T 150 - Dispersion mit einem weiß-trüben Farbton erhalten.

Mittels Fraunhofer-Beugung wurde der Mittelwert der Volumenverteilung zu D (4,3) = 0,50 µm bei einem Feinanteil der Verteilung 98,5 % < 1,22 µm bestimmt.

### b) Herstellung eines Uvinul^{®} T 150-haltigen wäßrigen Trockenpulvers

Nach Sprühtrocknung der Dispersion erhielt man ein Trockenpulver mit einem Wirkstoffgehalt von 20,56 Gew.-% Uvinul^{®} T 150 (Gehaltsbestimmung erfolgte UV/VIS-spektroskopisch). Das Trockenpulver ließ sich erneut in VE-Wasser unter Bildung einer weiß-trüben Dispersion (Hydrosol) redispergieren.

Mittels Fraunhofer-Beugung wurde der Mittelwert der Volumenverteilung in der Re-Dispersion bestimmt zu D (4,3) = 0,65 µm bei einem Feinanteil der Verteilung von 97,1 % < 1,22 µm.

### Beispiel 2

### a) Herstellung einer Uvinul^{®} T 150-haltigen wäßrigen Dispersion

10 g Uvinul^{®} T 150 wurden in eine Lösung von 2 g Ascorbylpalmitat in 216 g Aceton eingerührt. Zur Ausfällung des Uvinul^{®} T 150 in kolloid-disperser Form wurde diese molekular-disperse Lösung bei 20°C einer Mischkammer zugeführt. Dort erfolgte die Vermischung wäßrigen Lösung von 16,88 g Kollidon 90 F in 1500 ml VE-Wasser. Der gesamte Prozeß erfolgte unter Druckbegrenzung auf 40 bar, um eine Verdampfung des Lösemittels zu verhindern. Nach dem Mischen wurde eine kolloiddisperse Uvinul^{®} T 150-Dispersion mit einem weiß-trüben Farbton erhalten.

Durch quasi-elastische Lichtstreuung wurde die mittlere Teilchengröße zu 255 nm bei einer Verteilungsbreite von ± 40 % bestimmt. Mittels Fraunhofer-Beugung wurde der Mittelwert der Volumenverteilung zu D (4,3) = 0,62 µm bei einem Feinanteil der Verteilung 99,8 % < 1,22 µm bestimmt.

### b) Herstellung eines Uvinul^{®} T 150-haltigen wäßrigen Trockenpulvers

Sprühtrocknung der Dispersion nach 2a) führte zu einem nanopartikulären Trockenpulver. Der Wirkstoffgehalt im Pulver wurde UV/VIS-spektroskopisch zu 21,36 Gew.-% Uvinul^{®} T 150 bestimmt. Das Trockenpulver löst sich in VE-Wasser unter Bildung einer weiß-trüben Dispersion (Hydrosol).

Durch quasi-elastische Lichtstreuung wurde die mittlere Teilchengröße zu 350 nm bei einer Verteilungsbreite von ± 44 % bestimmt.
Mittels Fraunhofer-Beugung wurde der Mittelwert der Volumenverteilung bestimmt zu D (4,3) = 0,62 µm bei einem Feinanteil der Verteilung von 99,8 % < 1,22 µm.

### Beispiel 3

### Herstellung eines Uvinul® T 150-haltigen Trockenpulvers mit einem Wirkstoffgehalt von 20 Gew.-%

### a) Herstellung der wäßrigen Dispersion

10 g Uvinul^{®} T 150 wurden in einer Mischung aus 166 g Wasser und 50 g Aceton bei Raumtemperatur suspendiert und bei einer Temperatur von 240°C molekular gelöst. Zur Ausfällung des Uvinul^{®} T 150 in kolloid-disperser Form wurde die Lösung bei 240°C einer Mischkammer zugeführt. Dort erfolgte die Vermischung mit einer mittels 1 N NaOH auf pH 9,1 eingestellten wäßrigen Lösung von 16,88 g Gelatine B 100 Bloom und 11,25 g Lactose in 1500 ml VE-Wasser. Der gesamte Prozeß erfolgte unter Druckbegrenzung auf 40 bar, um eine Verdampfung des Lösemittels zu verhindern. Nach dem Mischen wurde eine kolloiddisperse Uvinul^{®} T 150 - Dispersion mit einem weiß-trüben Farbton erhalten.

Mittels Fraunhofer-Beugung wurde der Mittelwert der Volumenverteilung zu D (4,3) = 0,50 µm bei einem Feinanteil der Verteilung 98,5 % < 1,22 µm bestimmt.

### b) Herstellung eines Uvinul^{®} T 150-haltigen wäßrigen Trockenpulvers

Nach Sprühtrocknung der Dispersion erhielt man ein Trockenpulver mit einem Wirkstoffgehalt von 20,56 Gew.-% Uvinul^{®} T 150 (Gehaltsbestimmung erfolgte UV/VIS-spektroskopisch). Das Trockenpulver ließ sich erneut in VE-Wasser unter Bildung einer weiß-trüben Dispersion (Hydrosol) redispergieren.

Mittels Fraunhofer-Beugung wurde der Mittelwert der Volumenverteilung in der Re-Dispersion bestimmt zu D (4,3) = 0,65 µm bei einem Feinanteil der Verteilung von 97 % < 1,22 µm.

### Beispiel 4 (Vergleichsbeispiel)

### Herstellung eines Uvinul^{®} BMBM-haltigen Trockenpulvers mit einem Wirkstoffgehalt von 20 Gew.-%

### a) Herstellung der wäßrigen Dispersion

10 g Uvinul^{®} BMBM wurden in 216 g Aceton bei Raumtemperatur molekular-dispers gelöst. Zur Ausfällung des Uvinul^{®} BMBM in kolloid-disperser Form wurde die Lösung bei 20°C einer Mischkammer zugeführt. Dort erfolgte die Vermischung mit einer mittels 1 N NaOH auf pH 9,1 eingestellten wäßrigen Lösung von 16,88 g Gelatine B 100 Bloom und 11,25 g Lactose in 1500 ml VE-Wasser. Der gesamte Prozeß erfolgte unter Druckbegrenzung auf 40 bar, um eine Verdampfung des Lösemittels zu verhindern. Nach dem Mischen wurde eine kolloiddisperse Uvinul^{®} BMBM - Dispersion mit einem weiß-trüben Farbton erhalten.

Mittels Fraunhofer-Beugung wurde der Mittelwert der Volumenverteilung zu D (4,3) = 0,50 µm bei einem Feinanteil der Verteilung 98,5 % < 1,22 µm bestimmt.

### b) Herstellung eines Uvinul^{®} BMBM-haltigen Trockenpulvers

Nach Sprühtrocknung der Dispersion erhielt man ein Trockenpulver mit einem Wirkstoffgehalt von 20,56 Gew.-% Uvinul^{®} BMBM (Gehaltsbestimmung erfolgte UV/VIS-spektroskopisch). Das Trockenpulver ließ sich erneut in VE-Wasser unter Bildung einer weiß-trüben Dispersion (Hydrosol) redispergieren.

Mittels Fraunhofer-Beugung wurde der Mittelwert der Volumenverteilung in der Re-Dispersion bestimmt zu D (4,3) = 0,65 µm bei einem Feinanteil der Verteilung von 96,5 % < 1,20 µm.

### Beispiel 5

### Fällung von Uvinul^{®} T 150 und Blätterschellack als Hydrosol, mit Ascorbylpalmitat und Na-Caseinat

Als Wirkstoffsuspension wurden zunächst 23 g Uvinul^{®} T 150 und 47 g Blätterschellack in 343 g Isopropanol eingerührt. Als wäßrige Schutzkolloidlösung wurden 36 g Na-Caseinat und 1,1 g Ascorbylpalmitat in 10820 g Wasser gelöst. Der pH-Wert stellte sich dabei auf pH 6,9 ein.

Die Wirkstoffsuspension wurde auf eine Temperatur von 95°C aufgeheizt und mit einer Flußrate von 1,1 kg/h kontinuierlich mit Isopropanol/Wasser-Azeotrop, das auf eine Temperatur von 218°C gebracht wurde, bei einer Flußrate von 0,8 kg/h vermischt. Dabei lösten sich Uvinul^{®} T 150 und Blätterschellack molekular. Diese Lösung wurde mit der o.g. wäßrigen Schutzkolloidlösung, deren Flußrate 54,8 kg/h betrug, vermischt. Dabei wurden Uvinul^{®} T 150 und Blätterschellack als Matrixteilchen präzipitiert, die durch Na-Kaseinat als Hydrosol stabilisiert wurden. Das Hydrosol wurde anschließend in einem Rotationsverdampfer auf 31 % Feststoffgehalt aufkonzentriert, mit 14,6 g Gelatine B 200 Bloom aufgelackt und dann über Doppelemulgierung zu einem Trockenpulver aufgearbeitet. Der Gehalt an Uvinul^{®} T 150 im Trockenpulver betrug dann 15 %.

### Zubereitungen

### Beispiel 6

### Zusammensetzung für die Lippenpflege

### Massengehalt (Gew.-%)

| | |
|---|---|
| ad 100 | Eucerinum anhydricum |
| 10,00 | Glycerin |
| 10,00 | Titanium Dioxid, mikronisiert |
| 5,00 | Uvinul^{®} T 150 Trockenpulver aus Beispiel 1 |
| 8,00 | Octyl Methoxycinnamat |
| 5,00 | Zink Oxid |
| 4,00 | Castoröl |
| 4,00 | Pentaerythrithil Stearat/caprat/Caprylat Adipat |
| 3,00 | Glyceryl Stearat SE |
| 2,00 | Bienenwachs |
| 2,00 | Microkristallines Wachs |
| 2,00 | Quaternium-18 Bentonit |
| 1,50 | PEG-45/Dodecyl Glycol Copolymer |

### Beispiel 7

### Zusammensetzung für Sunblocker mit Mikropigmenten

### Massengehalt (Gew.-%)

| | |
|---|---|
| ad 100 | Wasser |
| 10,00 | Octyl Methoxcinnamat |
| 6,00 | PEG-7-Hydrogenated Castor Öl |
| 6,00 | Titanium Dioxid, mikronisiert |
| 5,00 | Uvinul^{®} T 150 Trockenpulver aus Beispiel 1 |
| 5,00 | Mineral Öl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Dimethicon |
| 0,50 | PEG-40-Hydrogenated Castor Öl |
| 0,50 | Tocopheryl Acetat |
| 0,50 | Phenoxyethanol |
| 0,20 | EDTA |

### Beispiel 8

### Fettfreies Gel

### Massengehalt (Gew.-%)

| | |
|---|---|
| ad 100 | Wasser |
| 8,00 | Octyl Methoxycinnamat |
| 7,00 | Titanium Dioxid, mikronisiert |
| 5,00 | Uvinul^{®} T 150 Trockenpulver aus Beispiel 1 |
| 5,00 | Glycerin |
| 5,00 | PEG-25 PABA |
| 1,00 | 4-Methylbenzyliden Campher |
| 0,40 | Acrylate C10-C30 Alkyl Acrylat Crosspolymer |
| 0,30 | Imidazolidinyl Urea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

### Beispiel 9

### Sonnencreme (LSF 20)

### Massengehalt (Gew.-%)

| | |
|---|---|
| ad 100 | Wasser |
| 8,00 | Octyl Methoxycinnamat |
| 8,00 | Titanium Dioxid, mikronisiert |
| 6,00 | PEG-7-Hydrogenated Castor Öl |
| 5,00 | Uvinul^{®} T 150 Trockenpulver aus Beispiel 1 |
| 6,00 | Mineral Öl |
| 5,00 | Zink Oxid |
| 5,00 | Isopropyl Palmitat |
| 0,30 | Imidazolidinyl Urea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

### Beispiel 10

### Sonnencreme wasserfest

### Massengehalt (Gew.-%)

| | |
|---|---|
| ad 100 | Wasser |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 5,00 | Uvinul^{®} T 150 Trockenpulver aus Beispiel 1 |
| 4,00 | Glycerin |
| 3,00 | Jojoba Öl |
| 2,00 | 4-Methylbenzyliden Campher |
| 2,00 | Titanium Dioxid, mikronisiert |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

### Beispiel 11

### Sonnenmilch (LSF 6)

### Massengehalt (Gew.-%)

| | |
|---|---|
| ad 100 | Wasser |
| 10,00 | Mineral Öl |
| 6,00 | PEG-7-Hydrogenated Castor Öl |
| 5,00 | Isopropyl Palmitat |
| 3,50 | Octyl Methoxycinnamat |
| 5,00 | Uvinul^{®} T 150 Trockenpulver aus Beispiel 1 |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0,60 | Magnesium Stearat |
| 0,50 | Tocopheryl Acetat |
| 3,00 | Glycerin |
| 0,25 | Methylparaben |
| 0,15 | Propylparaben |
| 0,05 | Tocopherol |

### Beispiel 12

### Tageslotion mit UV-Schutz

### Massengehalt (Gew.-%)

| | |
|---|---|
| ad 100 | Wasser |
| 2,00 | Cetearyl Alkohol |
| 1,00 | Glycerinmonostearat |
| 2,00 | Vaselin |
| 7,50 | Octyl Methoxycinnamat |
| 4,00 | Octyl Salicylat |
| 3,00 | Uvinul^{®} T 150 Trockenpulver aus Beispiel 1 |
| 1,50 | 4-tert.-Butyl-4'-methoxy-dibenzoylmethan |
| 0,50 | Dimethicon |
| 5,00 | Propylen Glycol |
| 0,20 | EDTA |
| 0,20 | Carbomer |
| 5,00 | C₁₂-C₁₅ Alkyl Benzoat |
| 0,27 | Triethanolamin |
| 1,00 | Tocopheryl Acetat |
| q.s. | Fragrance |

### Beispiel 13

### Tagescreme mit UV-Schutz

### Massengehalt (Gew.-%)

| | |
|---|---|
| ad 100 | Wasser |
| 2,00 | Cetearyl Alkohol |
| 2,00 | Cetyl Alkohol |
| 1,00 | Glycerinmonostearat |
| 2,00 | Vaselin |
| 7,50 | Octyl Methoxycinnamat |
| 4,00 | Octyl Salicylat |
| 3,00 | Uvinul^{®} T 150 Trockenpulver aus Beispiel 1 |
| 1,50 | 4-tert.-Butyl-4'-methoxy-dibenzoylmethan |
| 4,00 | Propylen Glycol |
| 0,20 | EDTA |
| 0,20 | Carbomer |
| 0,20 | Xanthan |
| 0,20 | C₁₀-C₃₀ Alkyl Acrylate Crosspolymer |
| 5,00 | C₁₂-C₁₅ Alkyl Benzoat |
| 0,54 | Triethanolamin |
| 1,00 | Tocopheryl Acetat |
| q.s. | Fragrance |
| q.s. | Konservierungsmittel |

### Beispiel 14

### Flüssiges Make Up

### Massengehalt (Gew.-%)

| | |
|---|---|
| ad 100 | Wasser |
| 2,00 | Cetearyl Alkohol |
| 2,00 | Ceteareth 25 |
| 6,00 | Glycerinmonostearat |
| 1,00 | Cetylalkohol |
| 8,00 | Paraffinöl |
| 7,00 | Cetearyl Octanoat |
| 0,2 | Dimethicon |
| 3,00 | Propylen Glycol |
| 1,00 | Panthenol |
| 3,00 | Uvinul^{®} T 150 Trockenpulver aus Beispiel 1 |
| 1,50 | 4-tert.-Butyl-4'-methoxy-dibenzoylmethan |
| 3,50 | Octyl Methoxycinnamat |
| 0,1 | Bisabolol |
| 5,70 | Titandioxid |
| 1,10 | Eisenoxid |
| q.s. | Fragrance |

### Beispiel 15

### Haargel mit Sonnenschutz

### Massengehalt (Gew.-%)

| | |
|---|---|
| ad 100 | Wasser |
| 1,20 | Carbomer |
| 0,50 | Hydroxyethyl Cellulose |
| 4,00 | Triethanolamin |
| 0,70 | PEG-40 Hydrogenated Castor oil |
| 1,50 | Uvinul^{®} T 150 Trockenpulver aus Beispiel 1 |
| 0,70 | 4-tert.-Butyl-4'-methoxy-dibenzoylmethan |
| 2,80 | Octyl Methoxycinnamat |
| 5,00 | Propylen Glycol |
| 0,01 | EDTA |
| q.s. | Fragrance |
| q.s. | Sicovit Patentblau 85 E 131 |

## Patentansprüche

1. Wäßrige Dispersionen schwer wasserlöslicher oder wasserunlöslicher organischer UV-Filtersubstanzen, **dadurch gekennzeichnet, daß** sie mindestens eine schwer wasserlösliche oder wasserunlösliche organische UV-Filtersubstanz als kolloid-disperse Phase in amorpher oder teilamorpher Form enthalten,
**dadurch** erhältlich, dass man
a) eine molekulardisperse Lösung mindestens einer schwer wasserlöslichen oder wasserunlöslichen organischen UV-Filtersubstanz in einem organischen Lösungsmittel herstellt,
b) diese Lösung mit einer wäßrigen Lösung mindestens eines Schutzkolloids versetzt, wobei die hydrophobe Phase der UV-Filtersubstanz als kolloid-disperse Phase entsteht und
c) die so erhaltene Dispersion vom organischen Lösungsmittel befreit,
worin die mindestens eine schwer wasserlösliche oder wasserunlösliche organische UV-Filtersubstanz ein 1,3,5-Triazinderivat der Formel I ist, 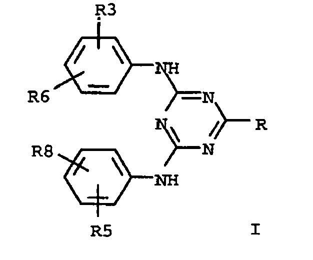 in der die Substituenten unabhängig voneinander folgende Bedeutung haben:
R Wasserstoff, Halogen, OH, C₁-C₂₀-Alkyl, C₁-C₂-Alkoxy, C₁-C₂₀- Alkoxyalkyl, C₁-C₂₀-Hydroxyalkoxy, NR¹R²,
oder einen Rest der Formel Ia 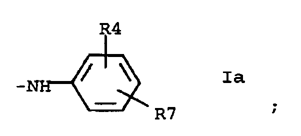
R¹ und R² Wasserstoff, C₁-C₂₀-Alkyl, gegebenenfalls mit einem oder mehreren C₁- C₄-Alkyl substituiertes C₆-C₁₂ Aryl, gegebenenfalls mit einem oder meh- reren C₁-C₄-Alkyl substituiertes C₇-C₁₀ Aralkyl, gegebenenfalls mit ei- nem oder mehreren C₁-C₄-Alkyl substituiertes Heteroaryl, C₅-C₈- Cycloalkyl;
R³ bis R⁵ Wasserstoff, OH, NR⁹R¹⁰, C₁-C₂₀-Alkoxy, gegebenenfalls mit einem oder mehreren C₁-C₄-Alkyl substituiertes C₆-C₁₂ Aryl, gegebenenfalls mit ei- nem oder mehreren C₁-C₄-Alkyl substituiertes C₇-C₁₀ Aralkyl, gegebe- nenfalls mit einem oder mehreren C₁-C₄-Alkyl substituiertes Heteroaryl, C₅-C₈-Cycloalkyl;
R⁶ bis R⁸ Wasserstoff, C₁-C₂₀-Alkoxy, -C(=O)-R¹¹,-C(=O)-X-R¹², SO₂R¹³, CN;
R⁹ bis R¹¹ Wasserstoff, C₁-C₂₀-Alkyl, gegebenenfalls mit einem oder mehreren C₁- C₄-Alkyl substituiertes C₆-C₁₂ Aryl, gegebenenfalls mit einem oder meh- reren C₁-C₄-Alkyl substituiertes C₇-C₁₀ Aralkyl, gegebenenfalls mit ei- nem oder mehreren C₁-C₄-Alkyl substituiertes Heteroaryl, C₅-C₈- Cycloalkyl;
R¹² Wasserstoff, C₁-C₂₀-Alkyl, gegebenenfalls mit einem oder mehreren C₁- C₄-Alkyl substituiertes C₆-C₁₂ Aryl, gegebenenfalls mit einem oder meh- reren C₁-C₄-Alkyl substituiertes C₇-C₁₀ Aralkyl, gegebenenfalls mit ei- nem oder mehreren C₁-C₄-Alkyl substituiertes Heteroaryl, C₅-C₈- Cycloalkyl oder einen Rest der Formel Sp-Sil;
R¹³ C₁-C₂₀-Alkyl, C₅-C₈-Cycloalkyl, gegebenenfalls mit einem oder mehre- ren C₁-C₄-Alkyl substituiertes C₆-C₁₂ Aryl, gegebenenfalls mit einem oder mehreren C₁-C₄-Alkyl substituiertes C₇-C₁₀ Aralkyl, gegebenenfalls mit einem oder mehreren C₁-C₄-Alkyl substituiertes Heteroaryl;
X O, NR¹⁴;
R¹⁴ Wasserstoff, C₁-C₂₀-Alkyl, gegebenenfalls mit einem oder mehreren C₁- C₄-Alkyl substituiertes C₆-C₁₂ Aryl, gegebenenfalls mit einem oder meh- reren C₁-C₄-Alkyl substituiertes C₁-C₁₀ Aralkyl, gegebenenfalls mit ei- nem oder mehreren C₁-C₄-Alkyl substituiertes Heteroaryl, C₅-C₈- Cycloalkyl;
Sp Spacer;
Sil Rest aus der Gruppe, bestehend aus Silane, Oligosiloxane und Polysilo- xane.

2. Wäßrige Dispersionen nach Anspruch 1, **dadurch gekennzeichnet, daß** es sich um Suspensionen handelt, die mindestens eine schwer wasserlösliche oder wasserunlösliche organische UV-Filtersubstanz als nanopartikuläre Teilchen mit einer mittleren Partikelgröße im Bereich von 0,01 bis 2 µm in amorpher oder teilamorpher Form enthalten.

3. Wäßrige Dispersionen nach Anspruch 1, **dadurch gekennzeichnet, daß** die Teilchen der kolloid-dispersen Phase einen Kern und eine Schale haben, wobei der Kern mindestens eine schwer wasserlösliche oder wasserunlösliche organische UV-Fiftersubstanz und die Schale mindestens ein Schutzkolloid enthält.

4. Wäßrige Dispersionen nach einem der Ansprüche 1 bis 3, enthaltend 0,1 bis 70 Gew.-% mindestens einer schwer wasserlöslichen oder wasserunlöslichen organischen UV-Filtersubstanz, 0,1 bis 80 Gew.-% mindestens eines polymeren Schutzkolloids, wobei sich alle Prozentangaben auf die Trockenmasse der wäßrigen Dispersion beziehen.

5. Wäßrige Dispersionen nach Anspruch 4, enthaltendzusätzlich 0,1 bis 70 Gew.-% mindestens eines Weichmachers, 0,01 bis 70 Gew.-% mindestens eines Emulgators und/oder 0,01 bis 50 Gew.-% mindestens eines Antioxidants und/oder Konservierungsmittels.

6. Wäßrige Dispersionen nach Anspruch 1, **dadurch gekennzeichnet, daß** die Teilchen der koliold-dispersen Phase eine wasserunlösliche Polymermatrix enthalten, in die mindestens eine schwer wasserlösliche oder wasserunlösliche organische UV-Filtersubstanz eingebettet ist.

7. Wäßrige Dispersionen nach Anspruch 6, **dadurch gekennzeichnet, daß** die kolloid-dispersen Teilchen zusätzlich von einem Schutzkolloid umhüllt sind.

8. Wäßrige Dispersionen nach Anspruch 6, enthaltend 0,1 bis 70 Gew.-% mindestens einer schwer wasserlöslichen oder wasserunlöslichen organischen UV-Filtersubstanz, 0,1 bis 80 Gew.-% mindestens eines wasserunlöslichen Matrixpolymers und 0 bis 80 Gew.-% mindestens eines polymeren Schutzkolloids, wobei sich alle Prozentangaben auf die Trockenmasse der wäßrigen Dispersion beziehen.

9. Wäßrige Dispersionen nach Anspruch 8, enthaltend zusätzlich 0,1 bis 70 Gew.-% mindestens eines Weichmachers, 0,01 bis 70 Gel.-% mindestens eines Emulgators und/oder 0,01 bis 50 Gew.-% mindestens eines Antioxidants.

10. Wäßrige Dispersionen nach Anspruch 1 bis 9, enthaltend mindestens ein 1,3,5-Triazinderivat der Formel Ib, in der die Substituenten unabhängig voneinander folgende Bedeutung haben: 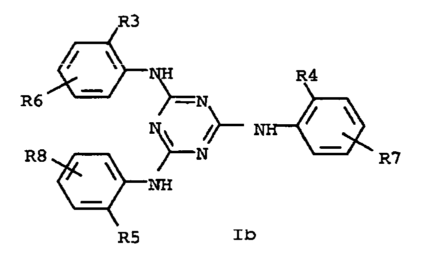
R³ bis R5 Wasserstoff, OH;
R⁶ bis R⁸ C₁-C₁₂-Alkoxy, -C(=O)-X-R₁₂;
X O, NR¹⁴;
R¹² und R¹⁴ Wasserstoff, C₄-C₈-Alkyl.

11. Verfahren zur Herstellung einer wäßrigen Dispersion mindestens einer schwer wasserlöslichen oder wasserunlöslichen organischen UV-Filtersubstanz, definiert gemäß Anspruch 1, **dadurch gekennzeichnet, daß** man
a) eine molekulardisperse Lösung mindestens einer schwer wasserlöslichen oder wasserunlöslichen organischen UV-Filtersubstanz in einem organischen Lösungsmittel herstellt,
b) diese Lösung mit einer wäßrigen Lösung mindestens eines Schutzkolloids versetzt, wobei die hydrophobe Phase der UV-Filtersubstanz ais kolloid-disperse Phase entsteht und
c) die so erhaltene Dispersion vom organischen Lösungsmittel befreit.

12. Verfahren nach Anspruch 11, **dadurch gekennzeichnet, daß** man als organisches Lösungsmittel im Verfahrensschritt a) mindestens ein mit Wasser mischbares, organisches Lösungsmittel oder eine Mischung aus Wasser und mindestens einem mit Wasser mischbaren organischen Lösungsmittel verwendet.

13. Verfahren nach einem der Ansprüche 11 oder 12, **dadurch gekennzeichnet, daß** man der molekulardispersen Lösung im Schritt a) weitere Zusatzstoffe, ausgewählt aus der Gruppe, bestehend aus Emulgatoren, Antioxidantien, Konservierungsmitteln, wasserunlöslichen Polymeren und kosmetischen Ölen zusetzt.

14. Verfahren nach einem der Ansprüche 11 bis 13, **dadurch gekennzeichnet, daß** im Schritt b) die wäßrige Schutzkolloidlösung zusätzlich mindestens einen Weichmacher enthält.

15. Verfahren nach einem der Ansprüche 11 bis 14, **dadurch gekennzeichnet, daß** man im Schritt a) die molekulardisperse Lösung von mindestens einer schwer wasserlöslichen oder wasserunlöslichen organischen UV-Filtersubstanz bei Temperaturen größer 15°C herstellt und unmittelbar anschließend im Schritt b) mit der wäßrigen Lösung des Schutzkolloids versetzt, wobei sich eine Mischungstemperatur von 35°C bis 120°C einstellt.

16. Verfahren zur Herstellung einer wäßrigen Dispersion mindestens einer schwer wasserlöslichen oder wasserunlöslichen organischen UV-Filtersubstanz, definiert gemäß Anspruch 6, **dadurch gekennzeichnet, daß** man
a) eine molekulardisperse Lösung mindestens einer schwer wasserlöslichen oder wasserunlöslichen organischen UV-Filtersubstanz und eines wasserunlöslichen Polymers in einem organischen Lösungsmittel herstellt,
b) diese Lösung mit einer wäßrigen Lösung mindestens eines Schutzkolloids versetzt, wobei die hydrophobe Phase der Mischung aus UV-Filtersubstanz und wasserunlöslichem Polymer als kolloid-disperse Phase entsteht und
c) die so erhaltene Dispersion vom organischen Lösungsmittel befreit.

17. Verfahren zur Herstellung eines Trockenpulvers, enthaltend mindestens eine schwer wasserlösliche oder wasserunlösliche organische UV-Filtersubstanz als nanopartikuläre Teilchen in amorpher oder teilamorpher Form, **dadurch gekennzeichnet, daß** man eine wäßrige Dispersion, definiert gemäß Anspruch 1, vom Wasser befreit und, gegebenenfalls in Gegenwart eines Überzugsmaterials, trocknet.

18. Pulverförmige Zubereitungen mindestens einer schwer wasserlöslichen oder wasserunlöslichen organischen Filtersubstanz enthaltend mindestens eine schwer wasserlöslichen oder wasserunlösliche organische UV-Filtersubstanz der Formel I gemäß Anspruch 1 als nanopartikuläre Teilchen mit einer mittleren Partikolgrörte im Bereich von 0,01 bis 2 µm in amorpher oder teilamorpher Form.

19. Verfahrenzur Herstellung einer mit Öl mischbaren Zubereitung mindestens einer schwer wasserlöslichen oder wasserunlöslichen organischen UV-Filtersubstanz in Form einer doppelten Dispersion, **dadurch gekennzeichnet, daß** man eine wäßrige Dispersion, definiert gemäß Anspruch 1 in Gegenwart eines Emulgators in Öl emulgiert.

20. Flüssige, mit Öl mischbare Zubereitungen mindestens einer schwer wasserlöslichen oder wasserunlöslichen organischen UV-Filtersubstanz der Formel I gemäß Anspruch 1, **dadurch gekennzeichnet, daß** sie als doppelte Dispersionssysteme eine wäßrig-disperse Phase mit einem Partikeldurchmesser kleiner 500 µm, in der schutzkolloid-stabilisierte Teilchen eines oder mehrerer schwer wasserlöslicher oder wasserunlöslicher organischer UV-Filtersubstanzen als nanopartikuläre Teilchen mit einer mittleren Partikelgröße im Bereich von 0,01 bis 2 µm in amorpher oder teilamorpher Form dispergiert vorliegen, in einem Öl als Dispersionsmittel enthalten.

21. Verwendung der wäßrigen Dispersionen, definiert gemäß einem der Ansprüche 1 bis 10 als photostabile Lichtschutzmittel für organische Materialien.

22. Verwendung der pulverförmige Zubereitungen, definiert gemäß Anspruch 18 als photostabile Lichtschutzmittel für organische Materialien.

23. Verwendung der flüssigen, mit Öl mischbaren Zubereitungen, definiert gemäß Anspruch 20 als photostabile Lichtschutzmittel für organische Materialien.

24. Verwendung nach einem der Ansprüche 21 bis 23 als Stabilisator für Kunststoffe und Lacke.

25. Verwendung nach einem der Ansprüche 21 bis 23 als photostabiler UV-Filter in kosmetischen und pharmazeutischen Zubereitungen zum Schutz der menschlichen Haut oder menschlicher Haare gegen UV-Strahlen.

26. Verwendung nach einem der Ansprüche 21 bis 23 als UV-Stabilisator in kosmetischen und pharmazeutischen Zubereitungen.

27. Lichtschutzmittel enthaltende kosmetische und pharmazeutische Zubereitungen zum Schutz der menschlichen Haut oder menschlicher Haare gegen UV-Licht im Bereich von 280 bis 400 nm, **dadurch gekennzeichnet, daß** sie in einem kosmetisch oder pharmazeutisch geeigneten Träger als photostabile UV-Filter wirksame Mengen einer wäßrigen Dispersion definiert gemäß Anspruch 1 enthalten.

28. Lichtschutzmittel enthaltende kosmetische und pharmazeutische Zubereitungen zum Schutz der menschlichen Haut oder menschlicher Haare gegen UV-Licht im Bereich von 280 bis 400 nm, **dadurch gekennzeichnet, daß** sie in einem kosmetisch oder pharmazeutisch geeigneten Träger als photostabile UV-Filter wirksame Mengen einer pulverförmigen Zubereitung, definiert gemäß Anspruch 18 enthalten.

29. Lichtschutzmittel enthaltende kosmetische und pharmazeutische Zubereitungen zum Schutz der menschlichen Haut oder menschlicher Haare gegen UV-Licht im Bereich von 280 bis 400 nm, **dadurch gekennzeichnet, daß** sie in einem kosmetisch oder pharmazeutisch geeigneten Träger als photostabile UV-Filter wirksame Mengen einer flüssigen, mit Öl mischbaren Zubereitungen, definiert gemäß Anspruch 20 enthalten.

## Claims

1. An aqueous dispersion of sparingly water-soluble or water-insoluble organic UV filter substances, which comprises at least one sparingly water-soluble or water-insoluble organic UV filter substance as colloidally disperse phase in amorphous or partially amorphous form,
obtainable by
a) preparing a molecularly disperse solution of at least one sparingly water-soluble or water-insoluble organic UV filter substance in an organic solvent,
b) treating this solution with an aqueous solution of at least one protective colloid, the hydrophobic phase of the UV filter substance forming as colloidally disperse phase, and
c) freeing the resulting dispersion from the organic solvent,
in which the at least one sparingly water-soluble or water-insoluble organic UV filter substance is a 1,3,5--triazine derivative of the formula I 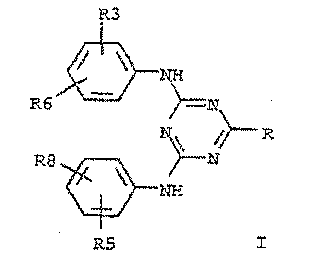 in which the substituents independently of one another have the following meanings:
R is hydrogen, halogen, OH, C₁-C₂₀-alkyl, C₁-C₂₀- alkoxy, C₁-C₂₀-alkoxyalkyl, C₁-C₂₀-hydroxyalkoxy, NR¹R² ,
or a radical of the formula Ia 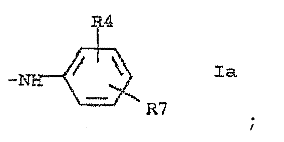
R¹ and R² are hydrogen, C₁-C₂₀-alkyl, C₆-C₁₂-aryl optionally substituted by one or more C₁-C₄- alkyl, C₇-C₁₀-aralkyl optionally substituted by one or more C₁-C₄-alkyl, heteroaryl optionally substituted by one or more C₁-C₄- alkyl, C₅-C₈-cycloalkyl;
R³ to R⁵ are hydrogen, OH, NR⁹R¹⁰, C₁-C₂₀-alkoxy, C₆-C₁₂- aryl optionally substituted by one or more C₁- C₄-alkyl, C₇-C₁₀-aralkyl optionally substituted by one or more C₁-C₄-alkyl, heteroaryl optionally substituted by one or more C₁-C₄- alkyl, C₅-C₈-cycloalkyl;
R⁶ to R⁸ are hydrogen, C₁-C₂₀-alkoxy, -C(=O)-R¹¹, - C (=O) -X-R¹², SO₂R¹³, CN;
R⁹ to R¹¹ are hydrogen, C₁-C₂₀-alkyl, C₆-C₁₂-aryl optionally substituted by one or more C₁-C₄- alkyl, C₇-C₁₀-aralkyl optionally substituted by one or more C₁-C₄-alkyl, heteroaryl optionally substituted by one or more C₁-C₄- alkyl, C₅-C₈-cycloalkyl;
R¹² is hydrogen, C₁-C₂₀-alkyl, C₆-C₁₂-aryl optionally substituted by one or more C₁-C₄- alkyl, C₇-C₁₀-aralkyl optionally substituted by one or more C₁-C₄-alkyl, heteroaryl optionally substituted by one or more C₁-C₄- alkyl, C₅-C₈-cycloalkyl or a radical of the formula Sp-Sil;
R¹³ is C₁-C₂₀-alkyl, C₅-C₈-cycloalkyl, C₆-C₁₂-aryl optionally substituted by one or more C₁-C₄- alkyl, C₇-C₁₀-aralkyl optionally substituted by one or more C₁-C₄-alkyl, heteroaryl optionally substituted by one or more C₁-C₄- alkyl;
X is O, NR¹⁴;
R¹⁴ is hydrogen, C₁-C₂₀-alkyl, C₆-C₁₂-aryl optionally substituted by one or more C₁-C₄- alkyl, C₇-C₁₀-aralkyl optionally substituted by one or more C₁-C₄-alkyl, heteroaryl optionally substituted by one or more C₁-C₄- alkyl, C₅-C₈-cycloalkyl;
Sp is a spacer;
Sil is a radical from the group consisting of silanes, oligosiloxanes and polysiloxanes.

2. The aqueous dispersion according to claim 1, which is a suspension which comprises at least one sparingly water-soluble or water-insoluble organic UV filter substance as nanoparticulate particles having a mean particle size in the range from 0.01 to 2 µm in amorphous or partially amorphous form.

3. The aqueous dispersion according to claim 1, wherein the particles of the colloidally disperse phase have a core and a shell, where the core comprises at least one sparingly water-soluble or water-insoluble organic UV filter substance, and the shell comprises at least one protective colloid.

4. The aqueous dispersion according to any of claims 1 to 3, comprising 0.1 to 70% by weight of at least one sparingly water-soluble or water-insoluble organic UV filter substance, 0.1 to 80% by weight of at least one polymeric protective colloid, all of the percentages being based on the dry mass of the aqueous dispersion.

5. The aqueous dispersion according to claim 4, additionally comprising 0.1 to 70% by weight of at least one softener, 0.01 to 70% by weight of at least one emulsifier and/or 0.01 to 50% by weight of at least one antioxidant and/or preservative.

6. The aqueous dispersion according to claim 1, wherein the particles of the colloidally disperse phase comprise a water-insoluble polymer matrix into which at least one sparingly water-soluble or water-insoluble organic UV filter substance has been embedded.

7. The aqueous dispersion according to claim 6, wherein the colloidally disperse particles have additionally been coated by a protective colloid.

8. The aqueous dispersion according to claim 6, comprising 0.1 to 70% by weight of at least one sparingly water-soluble or water-insoluble organic UV filter substance, 0.1 to 80% by weight of at least one water-insoluble matrix polymer and 0 to 80% by weight of at least one polymeric protective colloid, all of the percentages being based on the dry mass of the aqueous dispersion.

9. The aqueous dispersion according to claim 8, additionally comprising 0.1 to 70% by weight of at least one softener, 0.01 to 70% by weight of at least one emulsifier and/or 0.01 to 50% by weight of at least one antioxidant.

10. The aqueous dispersion according to claims 1 to 9, comprising at least one 1,3,5-triazine derivative of the formula Ib in which the substituents, independently of one another, have the following meanings: 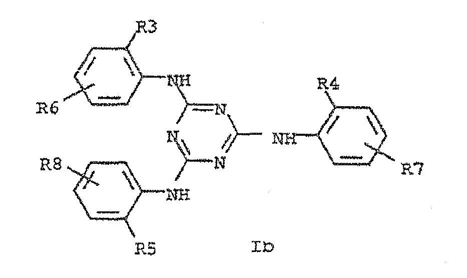
R³ t o R⁵ are hydrogen, OH;
R⁶ to R⁸ are C₁-C₁₂-alkoxy, -C (=O) -X-R¹²;
X is O, NR¹⁴;
R¹² and R¹⁴ are hydrogen, C₄-C₈-alkyl.

11. A process for the preparation of an aqueous dispersion of at least one sparingly water-soluble or water-insoluble organic UV filter substance, defined as in claim 1, which comprises
a) preparing a molecularly disperse solution of at least one sparingly water-soluble or water-insoluble organic UV filter substance in an organic solvent,
b) treating this solution with an aqueous solution of at least one protective colloid, the hydrophobic phase of the UV filter substance forming as colloidally disperse phase, and
c) freeing the resulting dispersion from the organic solvent.

12. The process according to claim 11, wherein the organic solvent used in process step a) is at least one water-miscible, organic solvent or a mixture of water and at least one water-miscible organic solvent.

13. The process according to either of claims 11 or 12, wherein further additives, chosen from the group consisting of emulsifiers, antioxidants, preservatives, water-insoluble polymers and cosmetic oils are added to the molecularly disperse solution in step a).

14. The process according to any of claims 11 to 13, wherein, in step b), the aqueous protective colloid solution additionally comprises at least one softener.

15. The process according to any of claims 11 to 14, wherein, in step a), the molecularly disperse solution of at least one sparingly water-soluble or water-insoluble organic UV filter substance is prepared at temperatures greater than 15°C, and immediately thereafter, is treated in step b) with the aqueous solution of the protective colloid, a mixing temperature of from 35°C to 120°C being established.

16. A process for the preparation of an aqueous dispersion of at least one sparingly water-soluble or water-insoluble organic UV filter substance, defined as in claim 6, which comprises
a) preparing a molecularly disperse solution of at least one sparingly water-soluble or water-insoluble organic UV filter substance and a water-insoluble polymer in an organic solvent,
b) treating this solution with an aqueous solution of at least one protective colloid, the hydrophobic phase of the mixture comprising UV filter substance and water-insoluble polymer forming as colloidally disperse phase, and
c) freeing the resulting dispersion from the organic solvent.

17. A process for the preparation of a dry powder comprising at least one sparingly water-soluble or water-insoluble organic UV filter substance as nanoparticulate particles in amorphous or partially amorphous form, which comprises freeing an aqueous dispersion defined as in claim 1 from the water and drying it, optionally in the presence of a coating material.

18. A pulverulent preparation of at least one sparingly water-soluble or water-insoluble organic filter substance comprising at least one sparingly water-soluble or water-insoluble organic UV filter substance of the formula I according to claim 1 as nanoparticulate particles having a mean particle size in the range from 0.01 to 2 µm in amorphous or partially amorphous form.

19. A process for the preparation of an oil-miscible preparation of at least one sparingly water-soluble or water-insoluble organic UV filter substance in the form of a double dispersion, which comprises emulsifying an aqueous dispersion defined as in claim 1 in the presence of an emulsifier in oil.

20. A liquid, oil-miscible preparation of at least one sparingly water-soluble or water-insoluble organic UV filter substance of the formula I according to claim 1, which, as a double dispersion system, comprises an aqueous-disperse phase having a particle diameter of less than 500 µm, in which protective-colloid-stabilized particles of one or more sparingly water-soluble or water-insoluble organic UV filter substances as nanoparticulate particles having a mean particle size in the range from 0.01 to 2 µm in amorphous or partially amorphous form are present in dispersed form, in an oil as dispersant.

21. The use of the aqueous dispersion defined as in any of claims 1 to 10 as a photostable light protection agent for organic materials.

22. The use of the pulverulent preparation defined as in claim 18 as a photostable light protection agent for organic materials.

23. The use of the liquid, oil-miscible preparation defined as in claim 20 as a photostable light protection agent for organic materials.

24. The use according to any of claims 21 to 23 as a stabilizer for plastics and coating materials.

25. The use according to any of claims 21 to 23 as a photostable UV filter in cosmetic and pharmaceutical preparations for protecting human skin or human hair from UV rays.

26. The use according to any of claims 21 to 23 as a UV stabilizer in cosmetic and pharmaceutical preparations.

27. A cosmetic or pharmaceutical preparation comprising a light protection agent for protecting the human skin or human hair from UV light in the range from 280 to 400 nm, which comprises, as photostable UV filter, an effective amount of an aqueous dispersion defined as in claim 1 in a cosmetically or pharmaceutically suitable carrier.

28. A cosmetic or pharmaceutical preparation comprising a light protection agent for protecting the human skin or human hair from UV light in the range from 280 to 400 nm, which comprises, as photostable UV filter, an effective amount of a pulverulent preparation defined as in claim 18 in
a cosmetically or pharmaceutically suitable carrier.

29. A cosmetic or pharmaceutical preparation comprising a light protection agent for the protection of human skin or human hair against UV light in the range from 280 to 400 nm, which comprises, as photostable UV filter, an effective amount of a liquid, oil-miscible preparation defined as in claim 20 in a cosmetically or pharmaceutically suitable carrier.

## Revendications

1. Dispersions aqueuses de substances organiques filtrant les UV, insolubles dans l'eau ou difficilement solubles dans l'eau, **caractérisées en ce qu'**elles contiennent sous forme amorphe ou partiellement amorphe au moins une substance organique filtrant les UV, insoluble dans l'eau ou difficilement soluble dans l'eau, en tant que phase en dispersion colloïdale,
pouvant être obtenues par
a) préparation d'une solution en dispersion moléculaire d'au moins une substance organique filtrant les UV, insoluble dans l'eau ou difficilement soluble dans l'eau, dans un solvant organique,
b) addition à cette solution d'une solution aqueuse d'au moins un colloïde protecteur, la phase hydrophobe de la substance filtrant les UV étant formée en tant que phase en dispersion colloïdale et
c) séparation de la dispersion ainsi obtenue d'avec le solvant organique,
dans lesquelles ladite au moins une substance organique filtrant les UV, insoluble dans l'eau ou difficilement soluble dans l'eau, est un dérivé de 1,3,5-triazine de formule I, 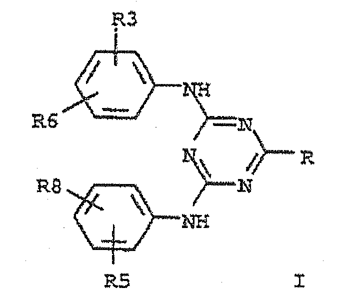 dans laquelle les substituants ont indépendamment les uns des autres les significations suivantes :
R représente un atome d'hydrogène ou d'halogène ou un groupe OH, alkyle en C₁-C₂₀, alcoxy en C₁-C₂₀, alcoxyalkyle en C₁-C₂₀, hydroxyalcoxy en C₁-C₂₀, NR¹R² ,
ou un radical de formule Ia 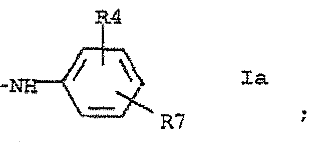
R¹ et R² représentent un atome d'hydrogène, un groupe alkyle en C₁-C₂₀, un radical aryle en C₆-C₁₂ éventuellement substitué par un ou plusieurs groupes alkyle en C₁-C₄, un radical aralkyle en C₇-C₁₀ éventuellement substitué par un ou plusieurs groupes alkyle en C₁-C₄, un radical hétéroaryle éventuellement substitué par un ou, plusieurs groupes alkyle en C₁-C₄, un radical cycloalkyle en C₅-C₈ ;
R³ à R⁵ représentent un atome d'hydrogène, OH, NR⁹R¹⁰, un groupe alcoxy en C₁-C₂₀, un radical aryle en C₆-C₁₂ éventuellement substitué par un ou plusieurs groupes alkyle en C₁-C₄, un radical aralkyle en C₇-C₁₀ éventuellement substitué par un ou plusieurs groupes alkyle en C₁-C₄, un radical hétéroaryle éventuellement substitué par un ou plusieurs groupes alkyle en C₁-C₄, un radical cycloalkyle en C₅-C₈ ;
R⁶ à R⁸ représentent un atome d'hydrogène, un groupe alcoxy en C₁-C₂₀, -C(=O)-R¹¹, -C (=O)-X-R¹², SO₂R¹³, CN;
R⁹ à R¹¹ représentent un atome d'hydrogène, un groupe alkyle en C₁-C₂₀, un radical aryle en C₆-C₁₂ éventuellement substitué par un ou plusieurs groupes alkyle en C₁-C₄, un radical aralkyle en C₇-C₁₀ éventuellement substitué par un ou plusieurs groupes alkyle en C₁-C₄, un radical hétéroaryle éventuellement substitué par un ou plusieurs groupes alkyle en C₁-C₄, un radical cycloalkyle en C₅-C₈ ;
R¹² représente un atome d'hydrogène, un groupe alkyle en C₁-C₂₀, un radical aryle en C₆-C₁₂ éventuellement substitué par un ou plusieurs groupes alkyle en C₁-C₄, un radical aralkyle en C₇-C₁₀ éventuellement substitué par un ou plusieurs groupes alkyle en C₁-C₄, un radical hétéroaryle éventuellement substitué par un ou plusieurs groupes alkyle en C₁-C₄, un radical cycloalkyle en C₅-C₈ ou un radical de formule Sp-Sil ;
R¹³ représente un groupe alkyle en C₁-C₂₀, un groupe cycloalkyle en C₅-C₈, un radical aryle en C₆-C₁₂ éventuellement substitué par un ou plusieurs groupes alkyle en C₁-C₄, un radical aralkyle en C₇-C₁₀ éventuellement substitué par un ou plusieurs groupes alkyle en C₁-C₄; un radical hétéroaryle éventuellement substitué par un ou plusieurs groupes alkyle en C₁-C₄ ;
X représente O, NR;
R¹⁴ représente un atome d'hydrogène, un groupe alkyle en C₁-C₂₀, un radical aryle en C₆-C₁₂ éventuellement substitué par un ou plusieurs groupes alkyle en C₁-C₄, un radical aralkyle en C₇-C₁₀ éventuellement substitué par un ou plusieurs groupes alkyle en C₁-C₄, un radical hétéroaryle éventuellement substitué par un ou plusieurs groupes alkyle en C₁-C₄, un radical cycloalkyle en C₅-C₈ ;
Sp représente un espaceur ;
Sil représente un radical choisi dans l'ensemble constitué par les silanes, organosilanes et polysiloxanes.

2. Dispersions aqueuses selon la revendication 1, **caractérisées en ce qu'**il s'agit de suspensions qui contiennent au moins une substance organique filtrant les UV, insoluble dans l'eau ou difficilement soluble dans l'eau, en particules nanoparticulaires ayant une taille moyenne de particule dans la plage de 0,01 à 2 µm, sous forme amorphe ou partiellement amorphe.

3. Dispersions aqueuses selon la revendication 1, **caractérisées en ce que** les particules de la phase en dispersion colloïdale comportent un noyau et une enveloppe, le noyau contenant au moins une substance organique filtrant les UV, insoluble dans l'eau ou difficilement soluble dans l'eau, et l'enveloppe contenant au moins un colloïde protecteur.

4. Dispersions aqueuses selon l'une quelconque des revendications 1 à 3, contenant de 0,1 à 70 % en poids d'au moins une substance organique filtrant les UV, insoluble dans l'eau ou difficilement soluble dans l'eau, de 0,1 à 80 % en poids d'au moins un colloïde protecteur polymère, toutes les données pondérales se rapportant à la matière sèche de la dispersion aqueuse.

5. Dispersions aqueuses selon la revendication 4, contenant en outre de 0,1 à 70 % en poids d'au moins un plastifiant, de 0,01 à 70 % en poids d'au moins un émulsifiant et/ou de 0,01 à 50 % en poids d'au moins un antioxydant et/ou conservateur.

6. Dispersions aqueuses selon la revendication 1, **caractérisées en ce que** les particules de la phase en dispersion colloïdale contiennent une matrice polymère insoluble dans l'eau, dans laquelle est incluse au moins une substance organique filtrant les UV, insoluble dans l'eau ou difficilement soluble dans l'eau.

7. Dispersions aqueuses selon la revendication 6, **caractérisées en ce que** les particules en dispersion colloïdale sont en outre enrobées d'un colloïde protecteur.

8. Dispersions aqueuses selon la revendication 6, contenant de 0,1 à 70 % en poids d'au moins une substance organique filtrant les UV, insoluble dans l'eau ou difficilement soluble dans l'eau, de 0,1 à 80 % en poids d'au moins un polymère-matrice insoluble dans l'eau et de 0 à 80 % en poids d'au moins un colloïde protecteur polymère, toutes les données pondérales se rapportant à la matière sèche de la dispersion aqueuse.

9. Dispersions aqueuses selon la revendication 8, contenant en outre de 0,1 à 70 % en poids d'au moins un plastifiant, de 0,01 à 70 % en poids d'au moins un émulsifiant et/ou de 0,01 à 50 % en poids d'au moins un antioxydant.

10. Dispersions aqueuses selon l'une quelconque des revendications 1 à 9, contenant au moins un dérivé de 1,3,5-triazine de formule Ib, dans laquelle les substituants ont indépendamment les uns des autres les significations suivantes : 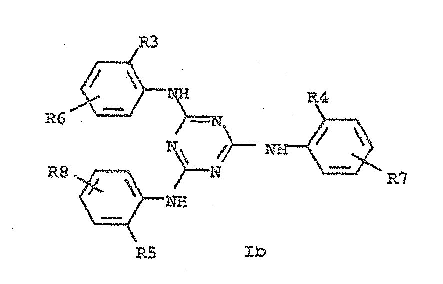
R³ à R⁵ représentent un atome d'hydrogène, OH ;
R⁶ à R⁸ représentent un groupe alcoxy en C₁-C₁₂, -C(=O)-X-R ;
X représente O, NR¹⁴ ;
R¹² et R¹⁴ représentent un atome d'hydrogène, un groupe alkyle en C₄-C₈.

11. Procédé pour la préparation d'une dispersion aqueuse d'au moins une substance organique filtrant les UV, insoluble dans l'eau ou difficilement soluble dans l'eau, définie selon la revendication 1, **caractérisé en ce que**
a) on prépare une solution en dispersion moléculaire d'au moins une substance organique filtrant les UV, insoluble dans l'eau ou difficilement soluble dans l'eau, dans un solvant organique,
b) on ajoute à cette solution une solution aqueuse d'au moins un colloïde protecteur, la phase hydrophobe de la substance filtrant les UV étant formée en tant que phase en dispersion colloïdale et
c) on sépare la dispersion ainsi obtenue d'avec le solvant organique.

12. Procédé selon la revendication 11, **caractérisé en ce que** dans l'étape a) du procédé on utilise comme solvant organique au moins un solvant organique miscible à l'eau ou un mélange d'eau et d'au moins un solvant organique miscible à l'eau.

13. Procédé selon la revendication 11 ou 12, **caractérisé en ce que** dans l'étape a) on ajoute à la solution en dispersion moléculaire d'autres additifs, choisis dans le groupe constitué par des émulsifiants, des antioxydants, des conservateurs, des polymères insolubles dans l'eau et des huiles cosmétiques.

14. Procédé selon l'une quelconque des revendications 11 à 13, **caractérisé en ce que** dans l'étape b) la solution aqueuse de colloïde protecteur contient en outre au moins un plastifiant.

15. Procédé selon l'une quelconque des revendications 11 à 14, **caractérisé en ce que** dans l'étape a) on prépare à des températures supérieures à 15 °C la solution en dispersion moléculaire d'au moins une substance organique filtrant les UV, insoluble dans l'eau ou difficilement soluble dans l'eau et immédiatement ensuite dans l'étape b) on y ajoute la solution aqueuse du colloïde protecteur, de sorte qu'il s'ajuste une température du mélange de 35 °C à 120 °C.

16. Procédé pour la préparation d'une dispersion aqueuse d'au moins une substance organique filtrant les UV, insoluble dans l'eau ou difficilement soluble dans l'eau, définie selon la revendication 6, **caractérisé en ce que**
a) on prépare une solution en dispersion moléculaire d'au moins une substance organique filtrant les UV, insoluble dans l'eau ou difficilement soluble dans l'eau, et d'un polymère insoluble dans l'eau, dans un solvant organique,
b) on ajoute à cette solution une solution aqueuse d'au moins un colloïde protecteur, la phase hydrophobe du mélange, consistant en substance filtrant les UV et polymère insoluble dans l'eau, étant formée en tant que phase en dispersion colloïdale et
c) on sépare la dispersion ainsi obtenue d'avec le solvant organique.

17. Procédé pour la préparation d'une poudre sèche, contenant au moins une substance organique filtrant les UV, insoluble dans l'eau ou difficilement soluble dans l'eau, en particules nanoparticulaires, sous forme amorphe ou partiellement amorphe, **caractérisé en ce qu'**on sépare d'avec l'eau une dispersion aqueuse, définie selon la revendication 1, et on la sèche éventuellement en présence d'une matière d'enrobage.

18. Préparations pulvérulentes d'au moins une substance organique filtrante, insoluble dans l'eau ou difficilement soluble dans l'eau, contenant au moins une substance organique filtrant les UV, insoluble dans l'eau ou difficilement soluble dans l'eau, de formule I selon la revendication 1, en particules nanoparticulaires ayant une taille moyenne de particule dans la plage de 0,01 à 2 µm, sous forme amorphe ou partiellement amorphe.

19. Procédé pour la production d'une préparation, miscible à l'huile, d'au moins une substance organique filtrant les UV, insoluble dans l'eau ou difficilement soluble dans l'eau, sous forme d'une double dispersion, **caractérisé en ce qu'**on émulsionne dans de l'huile une dispersion aqueuse, définie selon la revendication 1, en présence d'un émulsifiant.

20. Préparations liquides, miscibles à l'huile, d'au moins une substance organique filtrant les UV, insoluble dans l'eau ou difficilement soluble dans l'eau, de formule I selon la revendication 1, **caractérisées en ce qu'**en tant que systèmes de double dispersion elles contiennent une phase dispersée aqueuse, ayant un diamètre de particule inférieur à 500 µm, dans laquelle se trouvent dispersées des particules stabilisées par un colloïde protecteur, d'une ou plusieurs substances organiques filtrant les UV, insolubles dans l'eau ou difficilement solubles dans l'eau, en particules nanoparticulaires ayant une taille moyenne de particule dans la plage de 0,01 à 2 µm, sous forme amorphe ou partiellement amorphe, dans une huile en tant que dispersant.

21. Utilisation des dispersions aqueuses, définies selon l'une quelconque des revendications 1 à 10, en tant que photoprotecteurs photostables pour matières organiques.

22. Utilisation des préparations pulvérulentes, définies selon la revendication 18, en tant que photoprotecteurs photostables pour matières organiques.

23. Utilisation des préparations liquides, miscibles à l'huile, définies selon la revendication 20, en tant que photoprotecteurs photostables pour matières organiques.

24. Utilisation selon l'une quelconque des revendications 21 à 23, en tant que stabilisant pour matières plastiques et peintures.

25. Utilisation selon l'une quelconque des revendications 21 à 23, en tant que filtre UV photostable dans des préparations cosmétiques et pharmaceutiques pour la protection de la peau humaine ou des cheveux humains contre les rayons UV.

26. Utilisation selon l'une quelconque des revendications 21 à 23, en tant que stabilisant UV dans des préparations cosmétiques et pharmaceutiques

27. Préparations cosmétiques et pharmaceutiques contenant des photoprotecteurs, pour la protection de la peau humaine ou des cheveux humains contre la lumière UV dans la région de 280 à 400 nm, **caractérisées en ce qu'**elles contiennent en tant que filtres UV photostables des quantités efficaces d'une dispersion aqueuse définie selon la revendication 1, dans un véhicule cosmétiquement ou pharmaceutiquement convenable.

28. Préparations cosmétiques et pharmaceutiques contenant des photoprotecteurs, pour la protection de la peau humaine ou des cheveux humains contre la lumière UV dans la région de 280 à 400 nm, **caractérisées en ce qu'**elles contiennent en tant que filtres UV photostables des quantités efficaces d'une préparation pulvérulente définie selon la revendication 18, dans un véhicule cosmétiquement ou pharmaceutiquement convenable.

29. Préparations cosmétiques et pharmaceutiques contenant des photoprotecteurs, pour la protection de la peau humaine ou des cheveux humains contre la lumière UV dans la région de 280 à 400 nm, **caractérisées en ce qu'**elles contiennent en tant que filtres UV photostables des quantités efficaces d'une préparation liquide miscible à l'huile, définie selon la revendication 20, dans un véhicule cosmétiquement ou pharmaceutiquement convenable.
